(19) **Europäisches Patentamt / European Patent Office / Office européen des brevets**

(11) **EP 4 503 039 A1**

(12) **EUROPEAN PATENT APPLICATION**
published in accordance with Art. 153(4) EPC

(43) Date of publication:
05.02.2025 Bulletin 2025/06

(21) Application number: 23812185.9

(22) Date of filing: 25.05.2023

(51) International Patent Classification (IPC):
*G16B 40/20* [(2019.01)]    *G16B 30/00* [(2019.01)]
*G16B 50/00* [(2019.01)]    *G16B 15/30* [(2019.01)]
*G16B 30/10* [(2019.01)]    *G16B 25/10* [(2019.01)]
*G16B 20/00* [(2019.01)]    *C12Q 1/6869* [(2018.01)]
*G06N 20/00* [(2019.01)]

(52) Cooperative Patent Classification (CPC):
C12Q 1/6869; G06N 20/00; G16B 15/30;
G16B 20/00; G16B 25/10; G16B 30/00;
G16B 30/10; G16B 40/20; G16B 50/00

(86) International application number:
PCT/KR2023/007219

(87) International publication number:
WO 2023/229411 (30.11.2023 Gazette 2023/48)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 27.05.2022  KR 20220065487
09.02.2023  KR 20230017668

(71) Applicant: Spidercore Inc.
Daejeon 34014 (KR)

(72) Inventors:
• SEO, Dong-jin
  Seoul 06539 (KR)
• KANG, Min-geun
  Daejeon 34053 (KR)
• HWANG, Gyeong-jo
  Seoul 04362 (KR)
• LEE, Ki-won
  Daejeon 34185 (KR)
• LEE, Dae-hwan
  Daejeon 34177 (KR)

(74) Representative: BCKIP Part mbB
MK1
Landsbergerstraße 98, 3.Stock
80339 München (DE)

(54) **METHOD AND SYSTEM FOR DETERMINING OPTIMAL CHEMICAL MODIFICATIONS FOR BASE SEQUENCE OF RNA THERAPEUTIC AGENT**

(57)    In a method for determining an optimal chemical modification for a nucleotide sequence of an RNA therapeutic, a sequence modification module acquires, as learning data, biological characteristics corresponding to when multiple chemical modifications are applied to multiple nucleotide sequences, creates an optimal chemical modification prediction model by repeatedly performing a process of randomly selecting, among the learning data, at least two sequences in which different chemical modifications are applied to the same nucleotide sequence, sequentially inputting the selected sequences into an artificial neural network, and training the artificial neural network to compare output values for the input sequences and output a higher value as the biological characteristics for the input sequence are better, and uses the model to determine, as an optimal chemical modification, a chemical modification showing the best biological characteristics when applied to the nucleotide sequence of the RNA therapeutic, among first to wth chemical modifications.

EP 4 503 039 A1

FIG. 1

**Description**

Technical Field

The present invention relates to the development of new drugs using RNA (ribonucleic acid), and more specifically, to a method and a system for determining an optimal chemical modification for the nucleotide sequence of an RNA therapeutic.

Background Art

DNA (deoxyribonucleic acid), which contains genetic information, is a double-helix polymer made of nucleotide monomers. The genetic information contained in DNA is expressed into a specific protein through a complex process.

The nucleotide sequence contained in DNA contains information about the amino acids that make up proteins. In order for a specific protein to be expressed, a transcription process proceeds in which a specific nucleotide sequence of DNA required for the expression of the specific protein is copied into mRNA (messenger ribonucleic acid), and a translation process proceeds in which the mRNA copied binds to a ribosome required for protein synthesis to synthesize amino acids matching the nucleotide sequence of the mRNA, thus forming a protein. The protein produced through these processes migrates to an appropriate location in the cell or outside the cell, and gene expression is ultimately completed.

At this time, if the activity of mRNA copied from DNA is reduced, gene expression can be interfered with. Likewise, drugs with a new mechanism that utilize RNA, which acts to transfer genetic information during protein formation in cells, are called RNA therapeutics.

These RNA therapeutics are emerging as a new solution to incurable diseases for which there are no existing therapeutic agents. Recently, studies have been actively conducted on RNA therapeutics that can treat incurable diseases such as genetic diseases, cardiovascular diseases, and cancer.

In general, in order to develop an RNA therapeutic for a specific disease, a process is required that identifies the mRNA involved in the synthesis of a protein that causes the specific disease, and then finds an RNA nucleotide sequence that can effectively lower the activity of the identified mRNA.

However, RNA therapeutics have the side effect of inhibiting the synthesis of normal proteins by lowering the activity of other off-target mRNAs, even though having the positive function of lowering the activity of target mRNA.

In addition, even for RNA therapeutics with the same nucleotide sequence, the therapeutic efficiency and *in vivo* stability of the RNA therapeutics vary when different chemical modifications are applied thereto. Therefore, in order to develop an RNA therapeutic, it is necessary to determine what chemical modification will be applied to the nucleotide sequence of the RNA therapeutic.

DISCLOSURE

Technical Problem

The present invention has been conceived from the aforementioned point of view, and an object of the present invention is to provide a method for designing an RNA therapeutic, which may provide a nucleotide sequence of an RNA therapeutic that can effectively modulate the activity of target mRNA while preventing the side effect of modulating the activity of other off-target mRNAs, and may provide an optimal chemical modification that may be applied to the nucleotide sequence of the RNA therapeutic to improve the therapeutic efficiency and *in vivo* stability of the RNA therapeutic.

Another object of the present invention is to provide a system for designing an RNA therapeutic, which may provide a nucleotide sequence of an RNA therapeutic that can effectively modulate the activity of target mRNA while preventing the side effect of modulating the activity of other off-target mRNAs, and may provide an optimal chemical modification that may be applied to the nucleotide sequence of the RNA therapeutic to improve the therapeutic efficiency and *in vivo* stability of the RNA therapeutic.

Technical Solution

To achieve the above-described object of the present invention, in a method for determining an optimal chemical modification for a nucleotide sequence of an RNA therapeutic according to one embodiment of the present invention, a sequence modification module acquires, as learning data, data stored in a chemical modification information database that stores values representing biological characteristics which correspond to when multiple chemical modifications are applied to multiple nucleotide sequences; the sequence modification module creates an optimal chemical modification prediction model by repeatedly performing a process of randomly selecting, among the learning data, at least two sequences in which different chemical modifications are applied to the same nucleotide sequence, sequentially inputting the selected sequences into an artificial neural network, and training the artificial neural network to compare output values for the at least two sequences input into the artificial neural network and output a higher value as the biological characteristics for the sequence input into the artificial neural network are better; the sequence modification module receives a nucleotide sequence of an RNA therapeutic for modulating an activity of a target mRNA (messenger ribonucleic acid) involved in development of a specific disease; and the sequence modification module uses the optimal chemical modification prediction model to determine, as an optimal chemical modification, a chemical modification showing the best biological characteristics when applied to the nucleotide sequence of the RNA therapeutic, among first to $w^{th}$ chemical modifications, wherein w is an integer of 2 or more.

To achieve the above-described object of the present invention, a system for determining an optimal chemical modification for a nucleotide sequence of an RNA therapeutic includes a sequence modification module configured to: acquire, as learning data, data stored in a chemical modification information database that stores values representing biological characteristics which correspond to when multiple chemical modifications are applied to multiple nucleotide sequences; and create an optimal chemical modification prediction model by repeatedly performing a process of randomly selecting, among the learning data, at least two sequences in which different chemical modifications are applied to the same nucleotide sequence, sequentially inputting the selected sequences into an artificial neural network, and training the artificial neural network to compare output values for the at least two sequences input into the artificial neural network and output a higher value as the biological characteristics for the sequence input into the artificial neural network are better. The sequence modification module, when receiving a nucleotide sequence of an RNA therapeutic for modulating an activity of a target mRNA (messenger ribonucleic acid) involved in development of a specific disease, uses the optimal chemical modification prediction model to determine, as an optimal chemical modification, a chemical modification showing the best biological characteristics when applied to the nucleotide sequence of the RNA therapeutic, among first to $w^{th}$ chemical modifications, wherein w is an integer of 2 or more.

Advantageous Effects

The system for designing an RNA therapeutic according to embodiments of the present invention may not only provide a nucleotide sequence of an RNA therapeutic that can maximize the therapeutic effect for a specific disease by lowering the activity of a target mRNA while effectively preventing side effects, but also provide an optimal chemical modification that may be applied to the nucleotide sequence of the RNA therapeutic agent to further improve the therapeutic efficiency and *in vivo* stability of the RNA therapeutic agent. Thus, the system may effectively design an RNA therapeutic that may have a high therapeutic effect, reduced side effects, and an improved *in vivo* stability.

**Brief Description of Drawings**

FIG. 1 shows an RNA therapeutic agent design system according to one embodiment of the present invention.
FIG. 2 illustrates an example of the operation of an off-target analysis module included in the RNA therapeutic agent design system shown in FIG. 1.
FIG. 3 illustrates an example of the operation of the sequence generation module included in the RNA therapeutic agent design system shown in FIG. 1.
FIG. 4 shows an RNA therapeutic agent design system according to one embodiment of the present invention.
FIG. 5 illustrates an example of the operation of the secondary structure prediction module included in the RNA therapeutic agent design system shown in FIG. 4.
FIG. 6 illustrates an example of the operation of the sequence modification module included in the RNA therapeutic agent design systems in FIGS. 1 and 4.

**Best Mode**

Hereinafter, preferred embodiments of the present invention will be described in more detail with reference to the attached drawings. The same reference numerals are used for the same components in the drawings, and repeated descriptions for the same components will be omitted.

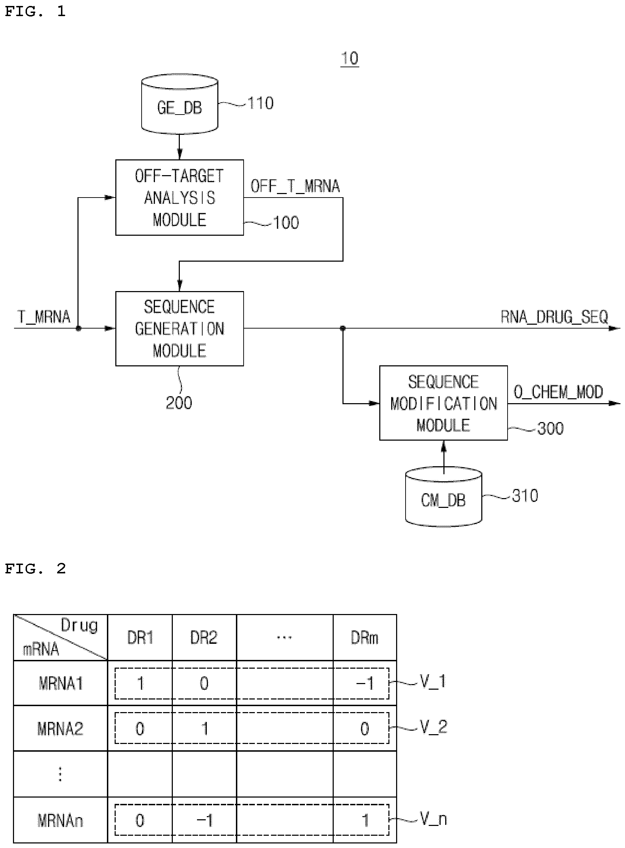

FIG. 1 shows an RNA therapeutic agent design system according to one embodiment of the present invention.

The RNA therapeutic agent design system 10 shown in FIG. 1 represents an RNA therapeutic agent design system that may provide not only a nucleotide sequence of an RNA therapeutic that can effectively modulate the activity of a target mRNA (messenger ribonucleic acid), but also an optimal chemical modification that may be applied to the nucleotide sequence of the RNA therapeutic to improve the therapeutic efficiency and *in vivo* stability of the RNA therapeutic.

Referring to FIG. 1, the RNA therapeutic agent design system 10 includes an off-target analysis module 100, a sequence generation module 200, and a sequence modification module 300.

The off-target analysis module 100 and the sequence generation module 200 receive, from the outside, a target mRNA (T_MRNA) involved in the synthesis of a protein that causes a specific disease.

When the off-target analysis module 100 receives the target mRNA (T_MRNA), the off-target analysis module 100 determines, as multiple off-target mRNAs (OFF_T_MRNA), mRNAs having gene expression patterns similar to that of the target mRNA (T_MRNA), among multiple mRNAs contained in the human body.

In one embodiment, the off-target analysis module 100 may use a gene expression database (GE_DB) 110, which previously stores the gene expression pattern of each of the multiple mRNAs contained in the human body, to cluster the multiple mRNAs into mRNAs having similar gene expression patterns to obtain first to $k^{th}$ clusters. Here, k represents an integer of 2 or more.

Thereafter, the off-target analysis module 100 may determine, as multiple off-target mRNAs (OFF_T_MRNA), mRNAs included in the cluster to which the target mRNA (T_MRNA) belongs, among the first to $k^{th}$ clusters.

Here, the gene expression database 110 may be any database that stores the gene expression patterns of each of the multiple mRNAs contained in the human body.

For example, the gene expression database 110 may be Gene Expression Omnibus (GEO), LINCS-L1000, Connectivity Map (CMAP), Human Protein Atlas (HPA), GTEx and FANTOM5.

In one embodiment, the gene expression database 110 may previously store the extent to which the expression level of each of the multiple mRNAs is modulated by each of the first to $m^{th}$ drugs. Here, m represents an integer of 2 or more.

Here, the first to $m^{th}$ drugs may be any drugs known to modulate the expression levels of mRNAs contained in the human body.

In this case, the off-target analysis module 100 may read out, from the gene expression database 110, the extent to which the expression level of each of the multiple mRNAs is modulated by each of the first to $m^{th}$ drugs, and may generate an m-dimensional expression level vector containing the extent to which the expression level of each of the multiple mRNAs is modulated by each of the first to $m^{th}$ drugs.

In one embodiment, the off-target analysis module 100 may generate first to $n^{th}$ expression level vectors (V_1, V_2, ..., V_n) containing information about whether the expression level of each of the multiple mRNAs is significantly increased, or significantly decreased, or not significantly changed by each of the first to $m^{th}$ drugs.

Thereafter, the off-target analysis module 100 may cluster the multiple expression level vectors corresponding to the multiple mRNAs into groups of expression level vectors existing in adjacent positions to obtain k clusters, and may classify the multiple mRNAs into the first to $k^{th}$ clusters by classifying mRNAs corresponding to expression level vectors included in the same cluster into the same cluster.

In one embodiment, the off-target analysis module 100 may apply a K-means algorithm to the multiple expression level vectors corresponding to the multiple mRNAs to cluster the multiple expression level vectors into k clusters, thereby classifying the multiple mRNAs into the first to $k^{th}$ clusters.

In one embodiment, the off-target analysis module 100 may apply a Gaussian mixture model (GMM) algorithm to the multiple expression level vectors corresponding to the multiple mRNAs to cluster the multiple expression level vectors into k clusters, thereby classifying the multiple mRNAs into the first to $k^{th}$ clusters.

In one embodiment, the off-target analysis module 100 may apply a DBSCAN (Density Based Spatial Clustering of Applications with Noise) algorithm to the multiple expression level vectors corresponding to the multiple mRNAs to cluster the multiple expression level vectors into k clusters, thereby classifying the multiple mRNAs into the first to $k^{th}$ clusters.

In one embodiment, the off-target analysis module 100 may apply a mean shift algorithm to the multiple expression level vectors corresponding to the multiple mRNAs to cluster the multiple expression level vectors into k clusters, thereby classifying the multiple mRNAs into the first to $k^{th}$ clusters.

In one embodiment, the off-target analysis module 100 may apply an agglomerative hierarchical clustering algorithm to the multiple expression level vectors corresponding to the multiple mRNAs to cluster the multiple expression level vectors into k clusters, thereby classifying the multiple mRNAs into the first to $k^{th}$ clusters.

In some embodiments, the off-target analysis module 100 clusters the multiple expression level vectors into k clusters using other known clustering algorithms, thereby classifying the multiple mRNAs into the first to $k^{th}$ clusters.

In some embodiments, the off-target analysis module 100 may cluster the multiple expression level vectors into k clusters using a clustering model with a known artificial neural network, thereby classifying the multiple mRNAs into the first to $k^{th}$ clusters.

FIG. 2 illustrates an example of the operation of the off-target analysis module included in the RNA therapeutic agent design system shown in FIG. 1.

FIG. 2 illustratively shows the operation of the off-target analysis module 100 in the case where the gene expression database 110 stores the extent to which the expression level of each of the first to $n^{th}$ mRNAs (MRNA1, MRNA2, ..., MRNAn) contained in the human body is modulated by each of the first to m$^{th}$ drugs (DR1, DR2, ..., DRm). Here, n represents an integer of 2 or more

Referring to FIG. 2, the off-target analysis module 100 may read out, from the gene expression database 110, the extent to which the expression level of each of the first to n$^{th}$ mRNAs (MRNA1, MRNA2, ..., MRNAn) is modulated by each of the first to m$^{th}$ drugs (DR1, DR2, ..., DRm), and then generate first to n$^{th}$ expression level vectors (V_1, V_2, ..., V_n) containing the extent to which the expression level of each of the first to n$^{th}$ mRNAs (MRNA1, MRNA2, ..., MRNAn) is modulated by each of the first to m$^{th}$ drugs (DR1, DR2, ..., DRm).

Therefore, each of the first to n$^{th}$ expression level vectors (V_1, V_2, ..., V_n) may be an m-dimensional vector.

In one embodiment, the off-target analysis module 100 may generate first to n$^{th}$ expression level vectors (V_1, V_2, ..., V_n) containing information about whether the expression level of each of the first to n$^{th}$ mRNAs (MRNA1, MRNA2, ..., MRNAn) is significantly increased, or significantly decreased, or not significantly changed by each of the first to m$^{th}$ drugs (DR1, DR2, ..., DRm).

At this time, the off-target analysis module 100 may generate first to n$^{th}$ expression level vectors (V_1, V_2, ..., V_n) for each of first to n$^{th}$ mRNAs (MRNA1, MRNA2, ..., MRNAn) in the form of numeric vectors by assigning a first value when the expression level of a specific mRNA is significantly increased by a specific drug, assigning a second value when the expression level of the specific mRNA is significantly decreased by the specific drug, and assigning a third value when the expression level of the specific mRNA is not significantly changed by the specific drug.

FIG. 2 illustratively shows a case where the first value is '1', the second value is '-1', and the third value is '0'.

Thus, in the example illustrated in FIG. 2, the first expression level vector (V_1) generated for the first mRNA may be [1, 0, ..., -1], the second expression level vector (V_2) generated for the second mRNA may be [ 0, 1, ..., 0], and the n$^{th}$ expression level vector (V_n) generated for the n$^{th}$ mRNA may be [0, -1, ..., 1].

However, the present invention is not limited thereto, and the first value, the second value, and the third value may be defined as any other numbers instead of '1', '-1', and '0'.

Thereafter, the off-target analysis module 100 may apply various types of clustering algorithms, such as a K-means algorithm, a Gaussian mixture model (GMM) algorithm, a DBSCAN (Density Based Spatial Clustering of Applications with Noise) algorithm, a mean shift algorithm, and an agglomerative hierarchical clustering, to the first to n$^{th}$ expression level vectors (V_1, V_2, ..., V_n) corresponding to the first to n$^{th}$ mRNAs (MRNA1, MRNA2, ..., MRNAn), thereby clustering the first to n$^{th}$ expression level vectors (V_1, V_2, ..., V_n) into k clusters.

In some embodiments, the number of clusters clustered by the off-target analysis module 100 may be predefined or may be determined according to the administrator's input.

Since the K-means algorithm, the Gaussian mixture model (GMM) algorithm, the DBSCAN (Density Based Spatial Clustering of Applications with Noise) algorithm, the mean shift algorithm, and the agglomerative hierarchical clustering algorithm are widely known clustering algorithms, detailed description of the process in which the off-target analysis module 100 clusters the first to n$^{th}$ expression level vectors (V_1, V_2, ..., V_n) into k clusters by applying various types of clustering algorithms is omitted herein.

Thereafter, the off-target analysis module 100 may classify the multiple mRNAs into the first to k$^{th}$ clusters by classifying mRNAs corresponding to expression level vectors included in the same cluster into the same cluster.

The off-target analysis module 100 may classify the multiple mRNAs contained in the human body into the first to k$^{th}$ clusters, and then determine, as multiple off-target mRNAs (OFF_T_MRNA), mRNAs included in the cluster to which the target mRNA (T _MRNA) belongs, among the first to k$^{th}$ clusters.

For example, the off-target analysis module 100 may read out, from the gene expression database 110, the extent to which the expression level of the target mRNA (T_MRNA) is modulated by each of first to m$^{th}$ drugs (DR1, DR2, ..., DRm), and may generate an m-dimensional target expression level vector containing the extent to which the expression level of the target mRNA (T_MRNA) is modulated by each of first to m$^{th}$ drugs (DR1, DR2, ..., DRm).

In one embodiment, the off-target analysis module 100 may generate the m-dimensional target expression level vector containing information about whether the expression level of the target mRNA (T_MRNA) is significantly increased, or significantly decreased, or not significantly changed by each of the first to m$^{th}$ drugs (DR1, DR2, ..., DRm).

At this time, as described above with reference to FIG. 2, the off-target analysis module 100 may generate the target expression level vector for the target mRNA (T_MRNA) in the form of a numeric vector by assigning a first value when the expression level of the target mRNA (T_MRNA) is significantly increased by a specific drug, assigning a second value when the expression level of the target mRNA (T_MRNA) is significantly decreased by the specific drug, and assigning a third value when the expression level of the target mRNA (T _MRNA) is not significantly changed by the specific drug.

Thereafter, the off-target analysis module 100 may determine, as a target cluster, a cluster corresponding to the center closest to the target expression level vector among the centers of the first to k$^{th}$ clusters, and may determine the mRNAs, included in the target cluster, as multiple off-target mRNAs (OFF_T_MRNA) for the target mRNA (T_MRNA).

Therefore, the multiple off-target mRNAs (OFF_T_MRNA) may have gene expression patterns similar to that of the target mRNA (T_MRNA).

Referring again to FIG. 1, the off-target analysis module 100 may provide the multiple off-target mRNAs (OFF_T_MRNA), determined for the target mRNA (T_MRNA), to the sequence generation module 200.

As the extent to which a specific RNA therapeutic modulates the activity of the target mRNA (T_MRNA) involved in the synthesis of a protein that causes a specific disease increases, the therapeutic effect of RNA therapeutic against the specific disease increases. However, since the multiple off-target mRNAs (OFF_T_MRNA) have gene expression patterns similar to that of the target mRNA (T_MRNA), the specific RNA therapeutic is highly likely to cause side effects of interfering with the synthesis of normal proteins by also modulating the activity of the multiple off-target mRNAs (OFF_T_MRNA).

Therefore, based on the target mRNA (T_MRNA) received from the outside and the multiple off-target mRNAs (OFF_T_MRNA) received from the off-target analysis module 100, the sequence generation module 200 determines the nucleotide sequence of an RNA therapeutic (RNA_DRUG_SEQ) that modulates the activity of the target mRNA (T_MRNA) to a great extent and modulates the activity of the multiple off-target mRNAs (OFF_T_MRNA) to a small extent.

Although the off-target analysis module 100 has been described above to determine, as multiple off-target mRNAs (OFF_T_MRNA), mRNAs having gene expression patterns similar to that of the target mRNA (T_MRNA) among the multiple mRNAs contained in the human body, and provide the determined off-target mRNAs to the sequence generation module 200, the present invention is not limited thereto, and in some embodiments, the sequence generation module 200 may determine, as multiple mRNAs (OFF_T_MRNA), all mRNAs contained in the human body, and perform the following operations.

In one embodiment, the sequence generation module 200 may perform reinforcement learning, thereby determining the nucleotide sequence of an RNA therapeutic (RNA_DRUG_SEQ) that modulate the activity of the target mRNA (T_MRNA) to a great extent and the activity of multiple off-target mRNAs (OFF_T_MRNA) to a small extent.

Specifically, the sequence generation module 200 may determine any candidate nucleotide sequence, and determine a reward for the candidate nucleotide sequence by increasing the reward as the extent to which the candidate nucleotide sequence modulates the activity of the target mRNA (T_MRNA) by binding to the target mRNA (T _MRNA) increases, and decreasing the reward as the extent to which the candidate nucleotide sequence modulates the activity of multiple off-target mRNAs (OFF_T_MRNA) by binding to each of the multiple off-target mRNAs (OFF_T_MRNA) increases.

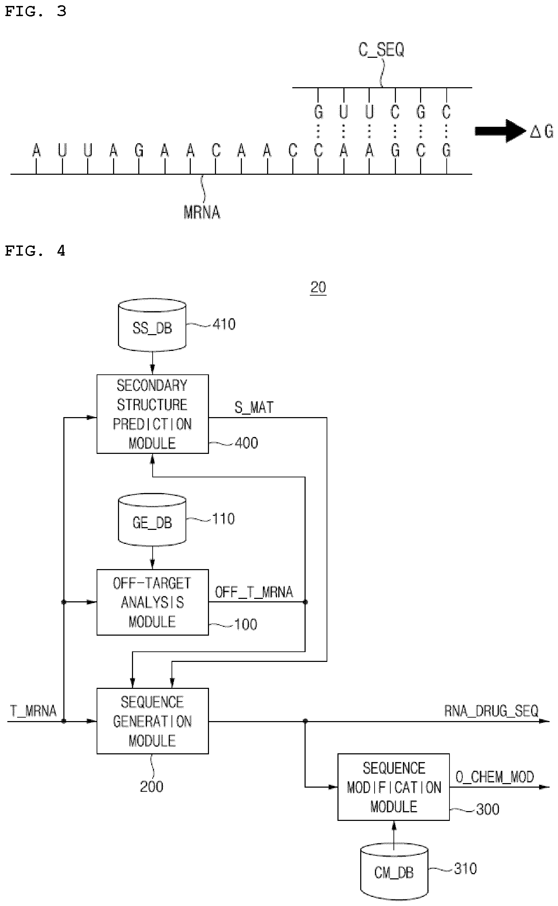

FIG. 3 illustrates an example of the operation of the sequence generation module included in the RNA therapeutic agent design system shown in FIG. 1.

FIG. 3 shows the nucleotide sequence of a specific mRNA (MRNA) and a candidate nucleotide sequence (C_SEQ).

As shown in FIG. 3, when the candidate nucleotide sequence (C_SEQ) binds to a specific mRNA (MRNA) by complementary base pairing between the nucleotide sequence of the specific mRNA (MRNA) and the candidate nucleotide sequence (C_SEQ), a certain amount of energy can be released.

As the amount of energy released when the candidate nucleotide sequence (C_SEQ) binds to the specific mRNA (MRNA) increases, the candidate nucleotide sequence (C_SEQ) becomes more stable by binding to the specific mRNA (MRNA), which means that the candidate nucleotide sequence (C_SEQ) easily binds to the specific mRNA (MRNA), thereby modulating the activity of the specific mRNA (MRNA) to a great extent.

Therefore, the greater the amount of energy released when the candidate nucleotide sequence (C_SEQ) binds to the target mRNA (T_MRNA), the extent to which the candidate nucleotide sequence modulates the activity of the target mRNA (T_MRNA) may be greater, and the greater the amount of energy released when the candidate nucleotide sequence (C_SEQ) binds to each of multiple off-target mRNAs (OFF_T_MRNA), the extent to which the candidate nucleotide sequence modulates the activity of each of the multiple off-target mRNAs (OFF_T_MRNA) may be greater.

Therefore, in one embodiment, the sequence generation module 200 may determine, as the reward, a value obtained by subtracting a value, obtained by multiplying the amount of energy released when the candidate nucleotide sequence (C_SEQ) binds to each of multiple off-target mRNAs (OFF_T_MRNA) by a weight, from the amount of energy released when the candidate nucleotide sequence (C_SEQ) binds to the target mRNA (T_MRNA).

More specifically, the amount of energy released when the candidate nucleotide sequence (C_SEQ) binds to a specific mRNA (MRNA) is related to the Gibbs free energy ($\Delta$G) for the reaction in which the candidate nucleotide sequence (C_SEQ) binds to a specific mRNA (MRNA).

Gibbs free energy ($\Delta$G) indicates the spontaneity of a chemical reaction. The greater the negative value of the Gibbs free energy of a chemical reaction, the greater the spontaneity, and the greater the positive value of the Gibbs free energy of a chemical reaction, the greater the non-spontaneity.

Therefore, the greater negative value of the Gibbs free energy ($\Delta$G) of the reaction in which the candidate nucleotide sequence (C_SEQ) binds to a specific mRNA (MRNA) may means that the binding of the candidate nucleotide sequence (C_SEQ) to the specific mRNA (MRNA) occurs more spontaneously so that the candidate nucleotide sequence (C_SEQ) modulates the activity of the specific mRNA (MRNA) to a great extent. At this time, the amount of energy released may also be large.

Accordingly, in one embodiment, the sequence generation module 200 may determine the reward for the candidate nucleotide sequence (C_SEQ) using Equation 1 below.

[Equation 1]

$$RW = -\Delta Gon + \alpha \sum \Delta Goff$$

wherein RW represents the reward, $\Delta$Gon represents the Gibbs free energy of the reaction in which the candidate nucleotide sequence (C_SEQ) binds to the target mRNA (T_MRNA), $\Delta$Goff represents the Gibbs free energy of the reaction in which the candidate nucleotide sequence (C_SEQ) binds to off-target mRNA (OFF_T_MRNA), and $\alpha$ represents the weight.

Therefore, the greater positive value of the reward for the candidate nucleotide sequence (C_SEQ) may mean that the extent to which the candidate nucleotide sequence (C_SEQ) modulates the activity of the target mRNA (T_MRNA) by binding to the target mRNA (T_MRNA) is greater, and the extent to which the candidate nucleotide sequence (C_SEQ) modulates the activity of each of multiple off-target mRNAs (OFF_T_MRNA) by binding to each of the off-target mRNAs (OFF_T_MRNA) is smaller.

Meanwhile, the sequence generation module 200 may previously store the Gibbs free energy for the binding reaction between various types of nucleotide sequences.

Thus, based on the Gibbs free energy for binding reactions between previously stored nucleotide sequences, the sequence generation module 200 may estimate the Gibbs free energy of the reaction in which the candidate nucleotide sequence (C_SEQ) binds to the target mRNA (T_MRNA) and the Gibbs free energy of the reaction in which the candidate nucleotide sequence (C_SEQ) binds to each of multiple off-target mRNAs (OFF_T_MRNA).

Referring again to FIG. 1, the sequence generation module 200 may perform the operation as described above with reference to FIG. 3 to determine the reward for the candidate nucleotide sequence, and then calculate the reward in the same manner as described above with reference to FIG. 3 while modifying the candidate nucleotide sequence in various ways, and learn about whether the reward for the candidate nucleotide sequence after modification increased or decreased compared to the reward for the candidate nucleotide sequence before modification.

For example, the sequence generation module 200 may learn about what modification for the candidate nucleotide sequence leads to an increase in the reward and what modification for the candidate nucleotide sequence leads to a decrease in the reward.

In this way, the sequence generation module 200 may calculate the reward while modifying the candidate nucleotide sequence in various ways, repeatedly perform the process of modifying the candidate nucleotide sequence in a direction that increases the reward, and determine the final candidate nucleotide sequence as the nucleotide sequence of the RNA therapeutic (RNA_DRUG_SEQ) if the reward is no longer increased through modification for the candidate nucleotide sequence.

Therefore, the nucleotide sequence of the RNA therapeutic (RNA_DRUG_SEQ) generated from the sequence generation module 200 can maximize the therapeutic effect for the specific disease by modulating the activity of the target mRNA (T_MRNA), and at the same time, effectively prevent side effects by minimizing the extent to which the RNA therapeutic modulates the activity of multiple off-target mRNAs (OFF_T_MRNA).

Meanwhile, the RNA therapeutic corresponding to the nucleotide sequence (RNA_DRUG_SEQ) generated from the sequence generation module 200 may be various types of RNA therapeutics.

In one embodiment, the RNA therapeutic corresponding to the nucleotide sequence (RNA_DRUG_SEQ) generated from the sequence generation module 200 may correspond to small interfering RNA (siRNA).

In one embodiment, the RNA therapeutic corresponding to the nucleotide sequence (RNA_DRUG_SEQ) generated from the sequence generation module 200 may correspond to an antisense oligonucleotide (ASO).

In one embodiment, the RNA therapeutic corresponding to the nucleotide sequence (RNA_DRUG_SEQ) generated from the sequence generation module 200 may correspond to an aptamer.

In one embodiment, the RNA therapeutic corresponding to the nucleotide sequence (RNA_DRUG_SEQ) generated from the sequence generation module 200 may correspond to CRISPR.

The nucleotide sequence of the RNA therapeutic (RNA_DRUG_SEQ) generated from the sequence generation module 200 may be provided to the sequence modification module 300.

The sequence modification module 300 uses a chemical modification information database (CM_DB) 310 that stores values representing biological properties corresponding to when multiple chemical modifications are applied to multiple nucleotide sequences, to determine, as an optimal chemical modification (O_CHEM_MOD), a chemical modification showing the highest therapeutic efficiency and *in vivo* stability when applied to the nucleotide sequence of the RNA therapeutic (RNA_DRUG_SEQ), among previously known first to wth (w is an integer of 2 or more) chemical modifications.

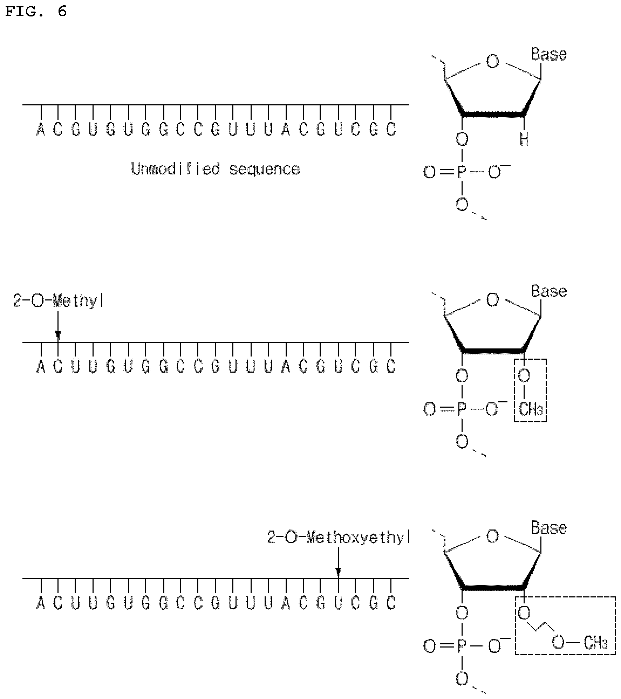

The detailed operation of the sequence modification module 300 to determine the optimal chemical modification (O_CHEM_MOD) will be described later with reference to FIG. 6.

FIG. 4 shows an RNA therapeutic agent design system according to one embodiment of the present invention.

Referring to FIG. 4, the RNA therapeutic agent design system 20 includes an off-target analysis module 100, a sequence generation module 200, a sequence modification module 300, and a secondary structure prediction module 400.

The RNA therapeutic agent design system 20 shown in FIG. 4 is the same as the RNA therapeutic agent design system 10 shown in FIG. 1, except that the RNA therapeutic agent design system 20 shown in FIG. 4 further includes the secondary structure prediction module 400.

Since the configuration and operation of the RNA therapeutic agent design system 10 shown in FIG. 1 have been described in detail with reference to FIGS. 1 to 3, redundant description will be omitted below, and only matters related to the secondary structure prediction module 400 in the RNA therapeutic agent design system 20 shown in FIG. 4 will be described in detail.

When the off-target analysis module 100 receives a target mRNA (T_MRNA), the off-target analysis module 100 determines, as multiple off-target mRNAs (OFF_T_MRNA), mRNAs having gene expression patterns similar to that of the target mRNA (T_MRNA) among multiple mRNAs contained in the human body.

The off-target analysis module 100 included in the RNA therapeutic agent design system 20 shown in FIG. 4 may operate in the same manner as the off-target analysis module 100 included in the RNA therapeutic agent design system 10 shown in FIG. 1.

Since the operation of the off-target analysis module 100 included in the RNA therapeutic agent design system 10 shown in FIG. 1 has been described in detail with reference to FIGS. 1 to 3, detailed description of the off-target analysis module 100 will be omitted below.

In general, most mRNAs exist in a folded state with complex structures. Specifically, an mRNA strand folds itself and exists in a folded state by forming hydrogen bonds between complementary base pairs.

In one embodiment, the secondary structure prediction module 400 may receive a target mRNA (T_MRNA) from the outside.

In this case, the secondary structure prediction module 400 may predict the secondary structure in which the target mRNA (T_MRNA) is folded.

In one embodiment, the secondary structure prediction module 400 may predict the secondary structure of the target mRNA (T_MRNA) by using a secondary structure database (SS_DB) 410 that previously stores the folded secondary structures of multiple RNAs contained in the human body.

For example, the secondary structure database 410 may be an ArchiveII database, an RNAStrAlign database, etc.

In this case, the secondary structure prediction module 400 may perform learning using the data stored in the secondary structure database 410 as learning data, thereby creating a secondary structure prediction model that outputs the secondary structure of the input mRNA nucleotide sequence.

Thereafter, the secondary structure prediction module 400 may predict the secondary structure of the target mRNA (T_MRNA) by inputting the nucleotide sequence of the target mRNA (T_MRNA) into the secondary structure prediction model.

The secondary structure prediction module 400 may estimate the bonding relationship between the bases in the target mRNA (T _MRNA) by predicting the secondary structure in which the target mRNA (T_MRNA) is folded.

In one embodiment, after the secondary structure prediction module 400 predicts the secondary structure in which the target mRNA (T_MRNA) is folded, it may generate a square matrix (S_MAT) that has rows and columns corresponding to the length of the nucleotide sequence of the target mRNA (T_MRNA) and represents the bonding relationship between the bases in the target mRNA (T_MRNA), which is formed by the secondary structure in which the target mRNA (T_MRNA) is folded.

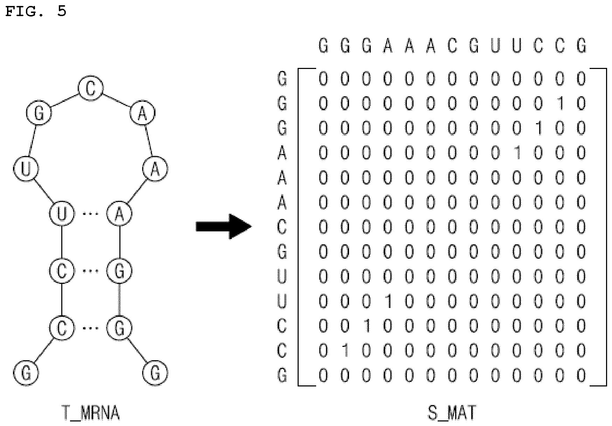

FIG. 5 illustrates an example of the operation of the secondary structure prediction module included in the RNA therapeutic agent design system shown in FIG. 4.

FIG. 5 illustratively shows that the target mRNA (T_MRNA) has a nucleotide sequence corresponding to G-G-G-A-A-A-C-G-U-U-C-C-G.

As shown in FIG. 5, the target mRNA (T_MRNA) may exist in a folded state by forming hydrogen bonds between complementary base pairs.

In the case of the target mRNA (T_MRNA) shown in FIG. 5, it is shown that the second base guanine and the twelfth base cytosine form a hydrogen bond with each other, the third base guanine and the eleventh base cytosine form a hydrogen bond with each other, and the fourth base adenine and the tenth base uracil form a hydrogen bond with each other.

In this case, the secondary structure prediction module 400 may generate a square matrix (S_MAT) that has rows and columns corresponding to the length of the nucleotide sequence of the target mRNA (T_MRNA) and represents the bonding relationship between the bases in the target mRNA (T_MRNA), which is formed by the secondary structure in which the target mRNA (T_MRNA) is folded.

Specifically, as shown in FIG. 5, the square matrix (S_MAT) generated by the secondary structure prediction module 400 may correspond to a symmetric matrix wherein, when the ith base and jth base in the nucleotide sequence of the target mRNA are bonded to each other by the secondary structure of the target mRNA (T_MRNA), the element corresponding to the ith row and jth column and the element corresponding to the jth row and ith column have a first value, and when the pth base and the qth base are not bonded to each other, the element corresponding to the pth row and qth column and the element corresponding to the qth row and pth column have a second value different from the first value. Here, i, j, p, and q represent natural numbers.

In one embodiment, as shown in FIG. 5, the first value may be '1' and the second value may be '0'.

Therefore, the square matrix (S_MAT) generated by the secondary structure prediction module 400 may represent the folded secondary structure of the target mRNA (T _MRNA).

Referring again to FIG. 4, the bonding relationship between the bases in the target mRNA (T_MRNA) , estimated by the secondary structure prediction module 400, may be provided to the sequence generation module 200.

Based on the bonding relationship between the bases in the target mRNA (T_MRNA), the sequence generation module 200 may determine the nucleotide sequence of the RNA therapeutic (RNA_DRUG_SEQ), which modulates the activity of the target mRNA (T_MRNA) to a great extent and modulates the activity of multiple off-target mRNAs (OFF_T_MRNA) to a small extent.

For example, the secondary structure prediction module 400 may provide a square matrix (S_MAT) generated for the target mRNA (T_MRNA) to the sequence generation module 200, and the sequence generation module 200 may determine, based on the square matrix (S_MAT) for the target mRNA (T_MRNA), the nucleotide sequence of the RNA therapeutic (RNA_DRUG_SEQ), which modulates the activity of the target mRNA (T _MRNA) to a great extent and modulates the activity of multiple off-target mRNAs (OFF_T_MRNA) to a small extent.

In one embodiment, the sequence generation module 200 may perform reinforcement learning based on the square matrix (S_MAT) for the target mRNA (T_MRNA), and determine the nucleotide sequence of the RNA therapeutic (RNA_DRUG_SEQ), which modulates the activity of the target mRNA (T_MRNA) to a great extent and modulates the activity of multiple off-target mRNAs (OFF_T_MRNA) to a small extent.

Hereinafter, description will be made of an example in which the sequence generation module 200 performs reinforcement learning and determines the nucleotide sequence of the RNA therapeutic (RNA_DRUG_SEQ).

As described above, mRNA generally exists in a folded state by forming hydrogen bonds between complementary base pairs.

Therefore, it is easier for a general RNA therapeutic to bind to a nucleotide sequence portion of an mRNA, in which the bases are not bonded to each other, than to a nucleotide sequence portion of the mRNA, in which the bases are bonded to each other.

Accordingly, in one embodiment, the sequence generation module 200 may determine the nucleotide sequence of the RNA therapeutic (RNA_DRUG_SEQ), on the assumption that the RNA therapeutic does not bind to a nucleotide sequence portion of the target mRNA (T_MRNA), in which the bases are boned to each other, but binds only to a nucleotide sequence portion of the target mRNA (T_MRNA), in which the bases are not boned to each other.

In this case, based on the square matrix (S_MAT) for the target mRNA (T_MRNA), the sequence generation module 200 may determine, as a binding portion to which the RNA therapeutic can bind, a sequence portion in which the bases are not bonded to each other, in the nucleotide sequence of the target mRNA (T_MRNA).

Thereafter, the sequence generation module 200 may determine any candidate nucleotide sequence, and determine a reward for the candidate nucleotide sequence by increasing the reward as the extent to which the candidate nucleotide sequence modulates the activity of the target mRNA (T_MRNA) by binding to the binding portion of the nucleotide sequence of the target mRNA (T_MRNA) increases, and decreasing the reward as the extent to which the candidate nucleotide sequence modulates the activity of each of multiple off-target mRNAs (OFF_T_MRNA) by binding to each of the multiple off-target mRNAs (OFF_T_MRNA) increases.

In one embodiment, the sequence generation module 200 may determine, as the reward, a value obtained by subtracting a value, obtained by multiplying the amount of energy released when the candidate nucleotide sequence binds to each of multiple off-target mRNAs (OFF_T_MRNA) by a weight, from the amount of energy released when the candidate nucleotide sequence binds to the binding portion of the nucleotide sequence of the target mRNA (T_MRNA).

For example, the sequence generation module 200 may determine the reward for the candidate nucleotide sequence using Equation 2 below.

[Equation 2]

$$RW = -\Delta Gon + \alpha \sum \Delta Goff$$

wherein RW represents the reward, $\Delta$Gon represents the Gibbs free energy of the reaction in which the candidate nucleotide sequence binds to the binding portion of the nucleotide sequence of the target mRNA (T_MRNA), $\Delta$Goff represents the Gibbs free energy of the reaction in which the candidate nucleotide sequence binds to the off-target mRNA (OFF_T_MRNA), and $\alpha$ represents the weight.

Therefore, the greater positive value of the reward for the candidate nucleotide sequence may mean that the extent to which the candidate nucleotide sequence modulates the activity of the target mRNA (T_MRNA) by binding to the target mRNA (T_MRNA) is greater, and the extent to which the candidate nucleotide sequence modulates the activity of each of multiple off-target mRNAs (OFF_T_MRNA) by binding to each of the off-target mRNAs (OFF_T_MRNA) is smaller.

The method in which the sequence generation module 200 included in the RNA therapeutic agent design system 20 shown in FIG. 4 calculates the reward for the candidate nucleotide sequence using Equation 2 above is identical to the method described above with reference to FIG. 3 in which the sequence generation module 200 included in the RNA therapeutic agent design system 10 shown FIG. 1 calculates the reward for the candidate nucleotide sequence using Equation 1 above.

Therefore, redundant description of the specific method in which the sequence generation module 200 calculates the reward for the candidate nucleotide sequence will be omitted below.

The sequence generation module 200 may perform the operation as described above to determine the reward for the candidate nucleotide sequence, and then calculate the reward in the same manner as described above while modifying the candidate nucleotide sequence in various ways, and learn about whether the reward for the candidate nucleotide sequence after modification increased or decreased compared to the reward for the candidate nucleotide sequence before modification.

For example, the sequence generation module 200 may learn about what modification for the candidate nucleotide sequence leads to an increase in the reward and what modification for the candidate nucleotide sequence leads to a decrease in the reward.

In this way, the sequence generation module 200 may calculate the reward while modifying the candidate nucleotide sequence in various ways, repeatedly perform the process of modifying the candidate nucleotide sequence in a direction that increases the reward, and determine the final candidate nucleotide sequence as the nucleotide sequence of the RNA therapeutic (RNA_DRUG_SEQ) if the reward is no longer increased through modification for the candidate nucleotide sequence.

Therefore, the nucleotide sequence of the RNA therapeutic (RNA_DRUG_SEQ), generated from the sequence generation module 200, can maximize the therapeutic effect for the specific disease by modulating the activity of the target mRNA (T_MRNA), and at the same time, effectively prevent side effects by minimizing the extent to which the RNA therapeutic modulates the activity of multiple off-target mRNAs (OFF_T_MRNA).

Hereinafter, description will be made of another example in which the sequence generation module 200 performs reinforcement learning to determine the nucleotide sequence of the RNA therapeutic (RNA_DRUG_SEQ).

As described above, mRNA generally exists in a folded state by forming hydrogen bonds between complementary base pairs.

Therefore, it is easier for a general RNA therapeutic to bind to a nucleotide sequence portion of an mRNA, in which the bases are not bonded to each other, than to a nucleotide sequence portion of the mRNA, in which the bases are bonded to each other.

Accordingly, in one embodiment, the sequence generation module 200 may determine the nucleotide sequence of the RNA therapeutic (RNA_DRUG_SEQ) in consideration of the fact that it is easier for the RNA therapeutic to bind to a nucleotide sequence portion of the target mRNA (T_MRNA), in which the bases are not boned to each other, than to a nucleotide sequence portion of the target mRNA (T_MRNA), in which the bases are boned to each other.

In this case, the sequence generation module 200 may determine any candidate nucleotide sequence, and regardless of the secondary structure of the target mRNA (T_MRNA), which is determined based on the square matrix (S_MAT) for the target mRNA (T_MRNA), it may calculate the reward by increasing the reward as the extent to which the candidate nucleotide sequence modulates the activity of the target mRNA (T_MRNA) by binding to the nucleotide sequence of the target mRNA (T_MRNA) increases, and decreasing the reward as the extent to which the candidate nucleotide sequence modulates the activity of each of multiple off-target mRNAs (OFF_T_MRNA) by binding to each of the multiple off-target mRNAs (OFF_T_MRNA) increases.

Meanwhile, since it is generally easier for an RNA therapeutic to bind to a nucleotide sequence portion of mRNA, in which the bases are not bonded to each other, than to a nucleotide sequence portion of mRNA, in which the bases are bonded to each other, it may be relatively difficult for the candidate nucleotide sequence to bind to the target mRNA (T_MRNA) as the proportion of bonded bases in the target mRNA (T_MRNA) among the bases of the target mRNA (T_MRNA) that binds to the candidate nucleotide sequence increases, and it may be relatively easy for the candidate nucleotide sequence to bind to the target mRNA (T_MRNA) as the proportion of bonded bases in the target mRNA (T_MRNA) among the bases of the target mRNA (T_MRNA) that binds to the candidate nucleotide sequence decreases.

Therefore, the sequence generation module 200 may determine, based on the square matrix (S_MAT) for the target mRNA (T_MRNA), a secondary structure penalty proportional to the proportion of bonded bases in the target mRNA (T_MRNA) among the bases of the target mRNA (T_MRNA) that binds to the candidate nucleotide sequence, and then determine the reward for the candidate nucleotide sequence by subtracting the secondary structure penalty from the calculated reward.

In one embodiment, the sequence generation module 200 may determine, as the reward, a value obtained by subtracting a value, obtained by multiplying the amount of energy released when the candidate nucleotide sequence binds to each of multiple off-target mRNAs (OFF_T_MRNA) by a weight, from the amount of energy released when the candidate nucleotide sequence binds to the nucleotide sequence of the target mRNA (T_MRNA), and further subtracting the secondary structure penalty.

For example, the sequence generation module 200 may determine the reward for the candidate nucleotide sequence using Equation 3 below.

[Equation 3]

$$RW = -\Delta Gon + \alpha \sum \Delta Goff - SP$$

wherein RW represents the reward, $\Delta$Gon represents the Gibbs free energy of the reaction in which the candidate nucleotide sequence binds to the nucleotide sequence of the target mRNA (T_MRNA), $\Delta$Goff represents the Gibbs free energy of the reaction in which the candidate nucleotide sequence binds to the off-target mRNA (OFF_T_MRNA), $\alpha$ represents the weight, and SP represents the secondary structure penalty.

Therefore, the greater positive value of the reward for the candidate nucleotide sequence may mean that the extent to which the candidate nucleotide sequence modulates the activity of the target mRNA (T_MRNA) by binding to the target mRNA (T_MRNA) is greater, and the extent to which the candidate nucleotide sequence modulates the activity of each of multiple off-target mRNAs (OFF_T_MRNA) by binding to each of the off-target mRNAs (OFF_T_MRNA) is smaller.

The method in which the sequence generation module 200 included in the RNA therapeutic agent design system 20 shown in FIG. 4 calculates the reward for the candidate nucleotide sequence using Equation 3 above is identical to the method described above with reference to FIG. 3 in which the sequence generation module 200 included in the RNA therapeutic agent design system 10 shown FIG. 1 calculates the reward for the candidate nucleotide sequence using Equation 1 above, except that the secondary structure penalty is subtracted from the reward.

Therefore, redundant description of the specific method in which the sequence generation module 200 calculates the reward for the candidate nucleotide sequence will be omitted below.

The sequence generation module 200 may perform the operation as described above to determine the reward for the candidate nucleotide sequence, and then calculate the reward in the same manner as described above while modifying the candidate nucleotide sequence in various ways, and learn about whether the reward for the candidate nucleotide sequence after modification increased or decreased compared to the reward for the candidate nucleotide sequence before modification.

For example, the sequence generation module 200 may learn about what modification for the candidate nucleotide sequence leads to an increase in the reward and what modification for the candidate nucleotide sequence leads to a decrease in the reward.

In this way, the sequence generation module 200 may calculate the reward while modifying the candidate nucleotide sequence in various ways, repeatedly perform the process of modifying the candidate nucleotide sequence in a direction that increases the reward, and determine the final candidate nucleotide sequence as the nucleotide sequence of the RNA therapeutic (RNA_DRUG_SEQ) if the reward is no longer increased through modification for the candidate nucleotide sequence.

Therefore, the nucleotide sequence of the RNA therapeutic (RNA_DRUG_SEQ), generated from the sequence generation module 200, can maximize the therapeutic effect for the specific disease by modulating the activity of the target mRNA (T_MRNA), and at the same time, effectively prevent side effects by minimizing the extent to which the RNA therapeutic modulates the activity of multiple off-target mRNAs (OFF_T_MRNA).

In another embodiment, as shown in FIG. 4, the secondary structure prediction module 400 may receive the target mRNA (T_MRNA) from the outside and receive multiple off-target mRNAs (OFF_T_MRNA) for the target mRNA (T_MRNA) from the off-target analysis module 100.

In this case, the secondary structure prediction module 400 may predict the secondary structure in which the corresponding mRNA is folded, for each of the target mRNA (T_MRNA) and the multiple off-target mRNAs (OFF_T_MRNA).

In one embodiment, the secondary structure prediction module 400 may predict the secondary structure of each of the target mRNA (T_MRNA) and the multiple off-target mRNAs (OFF_T_MRNA) by using a secondary structure database (SS_DB) 410 that previously stores the folded secondary structures of multiple RNAs contained in the human body.

For example, the secondary structure database 410 may be an ArchiveII database, an RNAStrAlign database, etc.

In this case, the secondary structure prediction module 400 may perform learning using the data stored in the secondary structure database 410 as learning data, thereby creating a secondary structure prediction model that outputs the secondary structure of the input mRNA nucleotide sequence.

Thereafter, the secondary structure prediction module 400 may predict the secondary structure of each of the target mRNA (T_MRNA) and the multiple off-target mRNAs (OFF_T_MRNA) by inputting the nucleotide sequence of each of the target mRNA (T_MRNA) and the multiple off-target mRNAs (OFF_T_MRNA) into the secondary structure prediction model.

The secondary structure prediction module 400 may estimate the bonding relationship between the bases in the target mRNA (T_MRNA) and the bonding relationship between the bases in each of the multiple off-target mRNAs (OFF_T_MRNA) by predicting the secondary structure in which each of the target mRNA (T_MRNA) and the multiple off-target mRNAs (OFF_T_MRNA) is folded.

In one embodiment, after the secondary structure prediction module 400 predicts the secondary structure in which each of the target mRNA (T_MRNA) and the multiple off-target mRNAs (OFF_T_MRNA) is folded, it may generate a square matrix (S_MAT) that has rows and columns corresponding to the length of the nucleotide sequence of the corresponding mRNA and represents the bonding relationship between the bases in the corresponding mRNA, which is formed by the secondary structure in which the corresponding mRNA is folded.

Specifically, the secondary structure prediction module 400 may predict the secondary structure in which the target mRNA (T_MRNA) is folded, and then generate a square matrix (S_MAT) that has rows and columns corresponding to the length of the nucleotide sequence of the target mRNA (T_MRNA) and represents the bonding relationship between the bases in the target mRNA (T_MRNA), which is formed by the secondary structure in which the target mRNA (T_MRNA) is folded.

In addition, the secondary structure prediction module 400 may predict, for each of the multiple off-target mRNAs (OFF_T_MRNA), the secondary structure in which the off-target mRNAs (OFF_T_MRNA) is folded, and generate a square matrix (S_MAT) that has rows and columns corresponding to the length of the nucleotide sequence of each of the off-target mRNAs (OFF_T_MRNA) and represents the bonding relationship between the bases in each of the off-target mRNAs (OFF_T_MRNA), which is formed by the secondary structure in which each of the off-target mRNAs (OFF_T_MRNA) is folded.

The secondary structure prediction module 400 may generate a square matrix (S_MAT) for the target mRNA (T_MRNA) and a square matrix (S_MAT) for each of the multiple off-target mRNAs (OFF_T_MRNA) in the same manner as described above with reference to FIG. 5.

The bonding relationship between the bases in the target mRNA (T_MRNA) and the bonding relationship between the bases in each of the multiple off-target mRNAs (OFF_T_MRNA), estimated by the secondary structure prediction module 400, may be provided to the sequence generation module 200.

The sequence generation module 200 may determine, based on the bonding relationship between the bases in the target mRNA (T_MRNA) and the bonding relationship between the bases in each of the multiple off-target mRNAs (OFF_T_MRNA), the nucleotide sequence of the RNA therapeutic (RNA_DRUG_SEQ), which modulates the activity of the target mRNA (T_MRNA) to a great extent and modulates the activity of the multiple mRNAs (OFF_T_MRNA) to a small extent.

For example, the secondary structure prediction module 400 may provide, to the sequence generation module 200, the square matrix (S_MAT) generated for the target mRNA (T_MRNA) and the square matrix (S_MAT) generated for each of the multiple off-target mRNAs (OFF_T_MRNA), and the sequence generation module 200 may determine, based on the square matrix (S_MAT) for the target mRNA (T_MRNA) and the square matrix (S_MAT) for each of the multiple off-target mRNAs (OFF_T_MRNA), the nucleotide sequence of the RNA therapeutic (RNA_DRUG_SEQ), which modulates the activity of the target mRNA (T_MRNA) to a great extent and modulates the activity of the multiple off-target mRNAs (OFF_T_MRNA) to a small extent.

In one embodiment, the sequence generation module 200 may perform reinforcement learning based on the square matrix (S_MAT) for each of the target mRNA (T_MRNA) and the multiple off-target mRNAs (OFF_T_MRNA), thereby determining the nucleotide sequence of the RNA therapeutic (RNA_DRUG_SEQ), which modulates the activity of the target mRNA (T_MRNA) in a great extent and modulates the activity of the multiple off-target mRNAs (OFF_T_MRNA) to a small extent.

Hereinafter, description will be made of an example in which the sequence generation module 200 performs reinforcement learning to determine the nucleotide sequence of the RNA therapeutic (RNA_DRUG_SEQ).

As described above, mRNA generally exists in a folded state by forming hydrogen bonds between complementary base pairs.

Therefore, it is easier for a general RNA therapeutic to bind to a nucleotide sequence portion of an mRNA, in which the bases are not bonded to each other, than to a nucleotide sequence portion of the mRNA, in which the bases are bonded to each other.

Accordingly, in one embodiment, the sequence generation module 200 may determine the nucleotide sequence of the RNA therapeutic (RNA_DRUG_SEQ), on the assumption that the RNA therapeutic does not bind to a nucleotide sequence portion of each of the target mRNA (T_MRNA) and the multiple off-target mRNAs (OFF_T_MRNA), in which the bases are boned to each other, but binds only to a nucleotide sequence portion of each of the target mRNA (T_MRNA) and the multiple off-target mRNAs (OFF_T_MRNA), in which the bases are not boned to each other.

In this case, based on the square matrix (S_MAT) for each of the target mRNA (T_MRNA) and the multiple off-target mRNAs (OFF_T_MRNA), the sequence generation module 200 may determine, as a binding portion to which the RNA therapeutic can bind, a sequence portion in which the bases are not bonded to each other, in each of the target mRNA (T_MRNA) and the multiple off-target mRNAs (OFF_T_MRNA).

Thereafter, the sequence generation module 200 may determine any candidate nucleotide sequence, and determine a reward for the candidate nucleotide sequence by increasing the reward as the extent to which the candidate nucleotide sequence modulates the activity of the target mRNA (T_MRNA) by binding to the binding portion of the nucleotide sequence of the target mRNA (T_MRNA) increases, and decreasing the reward as the extent to which the candidate nucleotide sequence modulates the activity of each of multiple off-target mRNAs (OFF_T_MRNA) by binding to the binding portion of the nucleotide sequence of each of the multiple off-target mRNAs (OFF_T_MRNA) increases.

In one embodiment, the sequence generation module 200 may determine, as the reward, a value obtained by subtracting a value, obtained by multiplying the amount of energy released when the candidate nucleotide sequence binds to the binding portion of the nucleotide sequence of each of multiple off-target mRNAs (OFF_T_MRNA) by a weight, from the amount of energy released when the candidate nucleotide sequence binds to the binding portion of the nucleotide sequence of the target mRNA (T_MRNA).

For example, the sequence generation module 200 may determine the reward for the candidate nucleotide sequence using Equation 4 below.

[Equation 4]

$$RW = -\Delta Gon + \alpha \sum \Delta Goff$$

wherein RW represents the reward, $\Delta Gon$ represents the Gibbs free energy of the reaction in which the candidate nucleotide sequence binds to the binding portion of the nucleotide sequences of the target mRNA (T_MRNA), $\Delta Goff$ the Gibbs free energy of the reaction in which the candidate nucleotide sequence binds to the binding portion of the nucleotide sequence of the off-target mRNA (OFF_T_MRNA), and $\alpha$ represents the weight.

Therefore, the greater positive value of the reward for the candidate nucleotide sequence may mean that the extent to which the candidate nucleotide sequence modulates the activity of the target mRNA (T_MRNA) by binding to the target mRNA (T_MRNA) is greater, and the extent to which the candidate nucleotide sequence modulates the activity of each of multiple off-target mRNAs (OFF_T_MRNA) by binding to each of the off-target mRNAs (OFF_T_MRNA) is smaller.

The method in which the sequence generation module 200 included in the RNA therapeutic agent design system 20 shown in FIG. 4 calculates the reward for the candidate nucleotide sequence using Equation 4 above is identical to the method described above with reference to FIG. 3 in which the sequence generation module 200 included in the RNA therapeutic agent design system 10 shown FIG. 1 calculates the reward for the candidate nucleotide sequence using Equation 1 above.

Therefore, redundant description of the specific method in which the sequence generation module 200 calculates the reward for the candidate nucleotide sequence will be omitted below.

The sequence generation module 200 may perform the operation as described above to determine the reward for the candidate nucleotide sequence, and then calculate the reward in the same manner as described above while modifying the candidate nucleotide sequence in various ways, and learn about whether the reward for the candidate nucleotide sequence after modification increased or decreased compared to the reward for the candidate nucleotide sequence before modification.

For example, the sequence generation module 200 may learn about what modification for the candidate nucleotide sequence leads to an increase in the reward and what modification for the candidate nucleotide sequence leads to a decrease in the reward.

In this way, the sequence generation module 200 may calculate the reward while modifying the candidate nucleotide sequence in various ways, repeatedly perform the process of modifying the candidate nucleotide sequence in a direction that increases the reward, and determine the final candidate nucleotide sequence as the nucleotide sequence of the RNA therapeutic (RNA_DRUG_SEQ) if the reward is no longer increased through modification for the candidate nucleotide sequence.

**EP 4 503 039 A1**

Therefore, the nucleotide sequence of the RNA therapeutic (RNA_DRUG_SEQ), generated from the sequence generation module 200, can maximize the therapeutic effect for the specific disease by modulating the activity of the target mRNA (T_MRNA), and at the same time, effectively prevent side effects by minimizing the extent to which the RNA therapeutic modulates the activity of multiple off-target mRNAs (OFF_T_MRNA).

Hereinafter, description will be made of another example in which the sequence generation module 200 performs reinforcement learning to determine the nucleotide sequence of the RNA therapeutic (RNA_DRUG_SEQ).

As described above, mRNA generally exists in a folded state by forming hydrogen bonds between complementary base pairs.

Therefore, it is easier for a general RNA therapeutic to bind to a nucleotide sequence portion of an mRNA, in which the bases are not bonded to each other, than to a nucleotide sequence portion of the mRNA, in which the bases are bonded to each other.

Accordingly, in one embodiment, the sequence generation module 200 may determine the nucleotide sequence of the RNA therapeutic (RNA_DRUG_SEQ) in consideration of the fact that it is easier for the RNA therapeutic to bind to a nucleotide sequence portion of each of the target mRNA (T _MRNA) and the multiple off-target mRNAs (OFF_T_MRNA), in which the bases are not boned to each other, than to a nucleotide sequence portion of each of the target mRNA (T_MRNA) and the multiple off-target mRNAs (OFF_T_MRNA), in which the bases are boned to each other.

In this case, the sequence generation module 200 may determine any candidate nucleotide sequence, and regardless of the secondary structure of the target mRNA (T _MRNA), which is determined based on the square matrix (S_MAT) for the target mRNA (T_MRNA), and the secondary structure of each of multiple off-target mRNAs (OFF_T_MRNA), which is determined based on the square matrix (S_MAT) for each of the multiple off-target mRNAs (OFF_T_MRNA), the sequence generation module 200 may calculate the reward by increasing the reward as the extent to which the candidate nucleotide sequence modulates the activity of the target mRNA (T_MRNA) by binding to the nucleotide sequence of the target mRNA (T_MRNA) increases, and decreasing the reward as the extent to which the candidate nucleotide sequence modulates the activity of each of multiple off-target mRNAs (OFF_T_MRNA) by binding to the nucleotide sequence of each of the multiple off-target mRNAs (OFF_T_MRNA) increases.

Meanwhile, since it is generally easier for an RNA therapeutic to bind to a nucleotide sequence portion of mRNA, in which the bases are not bonded to each other, than to a nucleotide sequence portion of mRNA, in which the bases are bonded to each other, it may be relatively difficult for the candidate nucleotide sequence to bind to the target mRNA (T_MRNA) as the proportion of bonded bases in the target mRNA (T_MRNA) among the bases of the target mRNA (T_MRNA) that binds to the candidate nucleotide sequence increases, and it may be relatively easy for the candidate nucleotide sequence to bind to the target mRNA (T _MRNA) as the proportion of bonded bases in the target mRNA (T_MRNA) among the bases of the target mRNA (T_MRNA) that binds to the candidate nucleotide sequence decreases.

Likewise, it may be relatively difficult for the candidate nucleotide sequence to bind to the off-target mRNA (OFF_T_MRNA) as the proportion of bonded bases in the off-target mRNA (OFF_T_MRNA) among the bases of the off-target mRNA (OFF_T_MRNA) that binds to the candidate nucleotide sequence increases, and it may be relatively easy for the candidate nucleotide sequence to bind to the off-target mRNA (OFF_T_MRNA) as the proportion of bonded bases in the off-target mRNA (OFF_T_MRNA) among the bases of the off-target mRNA (OFF_T_MRNA) that binds to the candidate nucleotide sequence decreases.

Therefore, the sequence generation module 200 may determine, based on the square matrix (S_MAT) for the target mRNA (T_MRNA), a target secondary structure penalty proportional to the proportion of bonded bases in the target mRNA (T_MRNA) among the bases of the target mRNA (T_MRNA) that binds to the candidate nucleotide sequence.

Likewise, the sequence generation module 200 may determine, based on the square matrix (S_MAT) for each of the off-target mRNAs (OFF_T_MRNA), an off-target secondary structure penalty proportional to the proportion of bonded bases in the off-target mRNA (OFF_T_MRNA) among the bases of the off-target mRNA (OFF_T_MRNA) that binds to the candidate nucleotide sequence.

Thereafter, the sequence generation module 200 may determine the reward for the candidate nucleotide sequence by subtracting the target secondary structure penalty for the target mRNA (T_MRNA) from the calculated reward and adding the off-target secondary structure penalty for each of the multiple off-target mRNAs (OFF_T_MRNA).

In one embodiment, the sequence generation module 200 may determine, as the reward, a value obtained by subtracting a value, obtained by multiplying the amount of energy released when the candidate nucleotide sequence binds to the nucleotide sequence of each of the multiple off-target mRNAs (OFF_T_MRNA) by a weight, from the amount of energy released when the candidate nucleotide sequence binds to the target mRNA (T_MRNA), further subtracting the target secondary structure penalty for the target mRNA (T _MRNA), and adding the off-target secondary structure penalty for each of the multiple off-target mRNAs (OFF_T _MRNA).

For example, the nucleotide sequence generation module 200 may determine the reward for the candidate nucleotide sequence using Equation 5 below.

15

[Equation 5]

$$RW = -\Delta Gon + \alpha \sum \Delta Goff - TSP + \sum OFFTSP$$

wherein RW represents the reward, ΔGon represents the Gibbs free energy of the reaction in which the candidate nucleotide sequence binds to the nucleotide sequence of the target mRNA (T_MRNA), ΔGoff represents the Gibbs free energy of the reaction in which the candidate nucleotide sequence binds to the nucleotide sequence of the off-target mRNA (OFF_T_MRNA), α represents the weight, TSP represents the target secondary structure penalty for the target mRNA (T_MRNA), and OFFTSP represents the off-target secondary structure penalty for the off-target mRNA (OFF_T_MRNA).

Therefore, the greater positive value of the reward for the candidate nucleotide sequence may mean that the extent to which the candidate nucleotide sequence modulates the activity of the target mRNA (T_MRNA) by binding to the target mRNA (T_MRNA) is greater, and the extent to which the candidate nucleotide sequence modulates the activity of each of the multiple off-target mRNAs (OFF_T_MRNA) by binding to each of the off-target mRNAs (OFF_T_MRNA) is smaller.

The method in which the sequence generation module 200 included in the RNA therapeutic agent design system 20 shown in FIG. 4 calculates the reward for the candidate nucleotide sequence using Equation 5 above is identical to the method described above with reference to FIG. 3 in which the sequence generation module 200 included in the RNA therapeutic agent design system 10 shown FIG. 1 calculates the reward for the candidate nucleotide sequence using Equation 1 above, except that the target secondary structure penalty for the target mRNA is subtracted from the reward and the off-target secondary structure penalty for each of the multiple off-target mRNAs (OFF_T_MRNA) is added.

Therefore, redundant description of the specific method in which the sequence generation module 200 calculates the reward for the candidate nucleotide sequence will be omitted below.

The sequence generation module 200 may perform the operation as described above to determine the reward for the candidate nucleotide sequence, and then calculate the reward in the same manner as described above while modifying the candidate nucleotide sequence in various ways, and learn about whether the reward for the candidate nucleotide sequence after modification increased or decreased compared to the reward for the candidate nucleotide sequence before modification.

For example, the sequence generation module 200 may learn about what modification for the candidate nucleotide sequence leads to an increase in the reward and what modification for the candidate nucleotide sequence leads to a decrease in the reward.

In this way, the sequence generation module 200 may calculate the reward while modifying the candidate nucleotide sequence in various ways, repeatedly perform the process of modifying the candidate nucleotide sequence in a direction that increases the reward, and determine the final candidate nucleotide sequence as the nucleotide sequence of the RNA therapeutic (RNA_DRUG_SEQ) if the reward is no longer increased through modification of the candidate nucleotide sequence.

Therefore, the nucleotide sequence of the RNA therapeutic (RNA_DRUG_SEQ), generated from the sequence generation module 200, can maximize the therapeutic effect for the specific disease by modulating the activity of the target mRNA (T_MRNA), and at the same time, effectively prevent side effects by minimizing the extent to which the RNA therapeutic modulates the activity of multiple off-target mRNAs (OFF_T_MRNA).

Meanwhile, the RNA therapeutic corresponding to the nucleotide sequence (RNA_DRUG_SEQ) generated from the sequence generation module 200 may be various types of RNA therapeutics.

In one embodiment, the RNA therapeutic corresponding to the nucleotide sequence (RNA_DRUG_SEQ) generated from the sequence generation module 200 may correspond to small interfering RNA (siRNA).

In one embodiment, the RNA therapeutic corresponding to the nucleotide sequence (RNA_DRUG_SEQ) generated from the sequence generation module 200 may correspond to an antisense oligonucleotide (ASO).

In one embodiment, the RNA therapeutic corresponding to the nucleotide sequence (RNA_DRUG_SEQ) generated from the sequence generation module 200 may correspond to an aptamer.

In one embodiment, the RNA therapeutic corresponding to the nucleotide sequence (RNA_DRUG_SEQ) generated from the sequence generation module 200 may correspond to CRISPR.

The nucleotide sequence of the RNA therapeutic (RNA_DRUG_SEQ) generated from the sequence generation module 200 may be provided to the sequence modification module 300.

The sequence modification module 300 uses a chemical modification information database (CM_DB) 310 that stores values representing biological characteristics corresponding to when multiple chemical modifications are applied to multiple nucleotide sequences, to determine, as an optimal chemical modification (O_CHEM_MOD), a chemical modification showing the highest therapeutic efficiency and *in vivo* stability when applied to the nucleotide sequence of the RNA therapeutic (RNA_DRUG_SEQ), among previously known first to $w^{th}$ (w is an integer of 2 or more) chemical modifications.

FIG. 6 illustrates an example of the operation of the sequence modification module included in each of the RNA therapeutic agent design systems shown in FIGS. 1 and 4.

The sequence modification module 300 included in the RNA therapeutic agent design system 10 shown in FIG. 1 and the sequence modification module 300 included in the RNA therapeutic agent design system 20 shown in FIG. 4 may operate in the same manner as described below with reference to FIG. 6.

As shown in FIG. 6, various types of chemical modifications may be applied to RNA nucleotide sequences contained in the human body.

FIG. 6 illustratively shows a molecular structural formula corresponding to when no chemical modification is applied to the RNA nucleotide sequence, a molecular structural formula corresponding to when a 2-O-methyl modification is applied to the sugar moiety at a specific position in the RNA nucleotide sequence, and a molecular structural formula corresponding to when a 2-O-methoxy-ethyl modification is applied to the sugar moiety at a specific position in the RNA nucleotide sequence.

However, the present invention is not limited thereto, and the first to $w^{th}$ chemical modifications considered by the sequence modification module 300 include any chemical modifications, such as 2-O-methyl, 2-O-methoxyethyl, 2-cyanoethyl, 5-methylcytidine, 2-Ome, 2-MOE, etc., which may be applied to the RNA nucleotide sequence.

Meanwhile, the chemical modification information database 310 may store values representing biological characteristics corresponding to when multiple chemical modifications are applied to multiple RNA nucleotide sequences contained in the human body.

Specifically, the chemical modification information database 310 may store a collection of values representing experimentally measured biological characteristics corresponding to when various types of chemical modifications are applied to each of a plurality of widely known RNA nucleotide sequences contained in the human body.

In one embodiment, the values representing the biological characteristics may include at least one of inhibition rate, which represents biological activity and efficacy, ED50, LD50, and IC50, which represent drug potency, ALT (alanine aminotransferase), AST (aspartate aminotransferase), bilirubin and creatinine levels, which represent drug toxicity, and elimination half-life.

For example, the chemical modification information database 310 may store a collection of data stored in the siRNAmod database, the PubChem database, etc.

In general, even the same RNA nucleotide sequence can exhibit different biological properties depending on the chemical modifications applied thereto.

Accordingly, the chemical modification information database 310 may store values representing the biological characteristics corresponding to when each of multiple chemical modifications is applied to multiple RNA nucleotide sequences contained in the human body.

In one embodiment, the sequence modification module 300 may previously store any chemical modifications, which are applicable to mRNAs contained in the human body, as the first to $w^{th}$ chemical modifications.

That is, the sequence modification module 300 may previously store, as the first to $w^{th}$ chemical modifications, any chemical modifications that may be applied to the sugar moiety, phosphate moiety, and base moiety in the nucleotide sequence of the RNA therapeutic (RNA_DRUG_SEQ).

Therefore, the first to $w^{th}$ chemical modifications may include at least one modification that is applied to a sugar moiety in the nucleotide sequence of the RNA therapeutic (RNA_DRUG_SEQ), at least one modification that is applied to a phosphate moiety in the nucleotide sequence of the RNA therapeutic (RNA_DRUG_SEQ), and at least one modification that is applied to a base moiety in the nucleotide sequence of the RNA therapeutic (RNA_DRUG_SEQ).

The sequence modification module 300 may train the artificial neural network using the data stored in the chemical modification information database 310 as learning data, thereby creating an optimal chemical modification prediction model that can determine a chemical modification showing the best biological characteristics when applied to the nucleotide sequence of the RNA therapeutic (RNA_DRUG_SEQ), among the first to $w^{th}$ chemical modifications.

Specifically, the sequence modification model 300 may create an optimal chemical modification prediction model by repeatedly performing a process of selecting, among the learning data, at least two sequences in which different chemical modifications are applied to the same nucleotide sequence, sequentially inputting the selected sequences into an artificial neural network, and training the artificial neural network to compare output values for the at least two sequences input into the artificial neural network and output a higher value as the biological characteristics for the sequence input into the artificial neural network are better;

Hereinafter, description will be made of an example of the process in which the sequence modification module 300 creates the optimal chemical modification prediction model by randomly selecting, among the learning data, two sequences in which different chemical modifications are applied to the same nucleotide sequence, and training the artificial neural network.

The sequence modification module 300 may randomly select, among the learning data, a pair of a first sequence and a second sequence in which different chemical modifications are applied to the same nucleotide sequence, sequentially input the first sequence and the second sequence into the artificial neural network, and acquire a first output value for the first sequence from the artificial neural network and a second output value for the second sequence from the artificial neural network.

Thereafter, the sequence modification module 300 may train the artificial neural network to increase a value obtained by subtracting the second output value from the first output value when the biological characteristics for the first sequence are better than the biological characteristics for the second sequence, and trains the artificial neural network to increase a value obtained by subtracting the first output value from the second output value when the biological characteristics for the second sequence are better than the biological characteristics for the first sequence.

The sequence modification module 300 may create the optimal chemical modification prediction model by randomly selecting, among the learning data, at least two sequences in which different chemical modifications are applied to the same nucleotide sequence, and repeatedly performing the above-described learning process on the artificial neural network using the at least two selected sequences.

Therefore, when a sequence to which a specific chemical modification is applied to a specific nucleotide sequence is input, the optimal chemical modification prediction model can output a greater value as the biological characteristics for the input sequence are better.

Although the description has been made above of an example of the process in which the sequence modification module 300 creates the optimal chemical modification prediction model by randomly selecting, among the learning data, two sequences in which different chemical modifications are applied to the same nucleotide sequence, and training the artificial neural network, a process in which the sequence modification module 300 creates the optimal chemical modification prediction model by randomly selecting at least three sequences in which different chemical modifications are applied to the same nucleotide sequence, inputting the selected sequences into the artificial neutral network, and training the artificial neutral network to compare output values for the at least three sequences may also be performed in the same manner.

Although it has been described above that the optimal chemical modification prediction model outputs a greater value as the biological characteristics for the input sequence are better, the present invention is not limited thereto.

In some embodiments, the nucleotide sequence modification module 300 may create the optimal chemical modification model by training the artificial neural network to output a smaller value as the biological characteristics for the sequence input into the artificial neural network are better.

In this case, the optimal chemical modification prediction model may output a smaller value as the biological characteristics for the input sequence are better.

In one embodiment, the artificial neural network may correspond to a Siamese neural network.

However, the present invention is not limited thereto, and the sequence modification module 300 may create the optimal chemical modification prediction model using various types of artificial neural networks.

Meanwhile, after the sequence modification module 300 creates the optimal chemical modification prediction model, as shown in FIGS. 1 to 4, the sequence modification module 300, when receives the nucleotide sequence of the RNA therapeutic (RNA_DRUG_SEQ) from the sequence generation module, may use the optimal chemical modification prediction model to determine, as an optimal chemical modification (O_CHEM_MOD), a chemical modification showing the best biological characteristics when applied to the nucleotide sequence of the RNA therapeutic (RNA_DRUG_SEQ), among the first to $w^{th}$ chemical modifications.

In addition, the sequence modification module 300 may provide the optimal chemical modification (O_CHEM_MOD) and a position in the nucleotide sequence of the RNA therapeutic (RNA_DRUG_SEQ), to which the optimal chemical modification (O_CHEM_MOD) is to be applied.

Hereinafter, description will be made of an example of the method in which the sequence modification module 300 uses the optimal chemical modification model to determine, as an optimal chemical modification (O_CHEM_MOD), a chemical modification showing the best biological characteristic when applied to the nucleotide sequence of the RNA therapeutic (O_CHEM_MOD), among the first to $w^{th}$ chemical modifications.

The sequence modification module 300 may generate multiple sequences by applying each of the first to $w^{th}$ chemical modifications to the nucleotide sequence of the RNA therapeutic (RNA_DRUG_SEQ).

For example, the sequence modification module 300 may generate sequences corresponding to the results of applying the first chemical modification to all positions of the nucleotide sequence of the RNA therapeutic (RNA_DRUG_SEQ), to which the first chemical modification is applicable, generate sequences corresponding to the results of applying the second chemical modification to all positions of the nucleotide sequence of the RNA therapeutic (RNA_DRUG_SEQ), to which the second chemical modification is applicable, and generate sequences corresponding to the results of applying the $w^{th}$ chemical modification to all positions of the nucleotide sequence of the RNA therapeutic (RNA_DRUG_SEQ), to which the $w^{th}$ chemical modification is applicable.

Accordingly, the number of the multiple sequences generated from the sequence modification module 300 may be much greater than w.

In addition, each of the multiple sequences may represent a molecular structure corresponding to each case in which each of the first to wth chemical modifications is applied to each of multiple applicable positions in the nucleotide sequence of the RNA therapeutic (RNA_DRUG_SEQ).

Meanwhile, it is general that biological characteristics are better when chemical modification is applied to the positions corresponding to the bases located at one end and the bases located at the other end of the nucleotide sequence of the RNA therapeutic (RNA_DRUG_SEQ) than when chemical modification is applied to the positions corresponding to the bases in the center of the nucleotide sequence of the RNA therapeutic (RNA_DRUG_SEQ).

Therefore, in some embodiments, the sequence modification module 300 may generate the multiple sequences by applying each of the first to wth chemical modifications to limited positions corresponding to the first a bases and the last b bases in the nucleotide sequence of the RNA therapeutic (RNA_DRUG_SEQ), wherein a and b are each an integer.

Thereafter, the sequence modification module 300 may sequentially input the multiple sequences into the optimal chemical modification prediction model, and acquire multiple output values for the multiple sequences from the optimal chemical modification prediction model.

The sequence modification module 300 may determine, as the optimal chemical modification (O_CHEM_MOD), a chemical modification applied to a sequence, which corresponds to the maximum value among the multiple output values, among the multiple sequences.

In addition, the sequence modification module 300 may provide the optimal chemical modification (O_CHEM_MOD) and a position in the nucleotide sequence of the RNA therapeutic (RNA_DRUG_SEQ), to which the optimal chemical modification (O_CHEM_MOD) is to be applied, based on the sequence corresponding to the maximum value among the maximum output values.

In some embodiments, the sequence modification module 300 may determine, as the optimal chemical modification (O_CHEM_MOD), all chemical modifications applied to sequences, which correspond to output values having a threshold value or more, among the multiple output values.

Hereinafter, description will be made of another example of the method in which the sequence modification module 300 uses the optimal chemical modification prediction model to determine, as the optimal chemical modification (O_CHEM_MOD), a chemical modification showing the best biological characteristic when applied to the nucleotide sequence of the RNA therapeutic (RNA_DRUG_SEQ), among the first to wth chemical modifications.

The sequence modification module 300 may generate multiple chemical modification combinations by combining one or more of the first to wth chemical modifications.

For example, the sequence modification module 300 may generate multiple chemical modification combinations by variously combining two or more chemical modifications among the first to wth chemical modifications.

Thereafter, the nucleotide sequence modification module 300 may generate a plurality of sequences by applying each of the plurality of chemical modification combinations to the nucleotide sequence (RNA_DRUG_SEQ) of the RNA therapeutic agent.

For example, the sequence modification module 300 may generate the multiple sequences by generating sequences corresponding to the results of applying the first chemical modification combination to all positions of the nucleotide sequence of the RNA therapeutic (RNA_DRUG_SEQ), to which two or more chemical modifications included in the first chemical modification combination are applicable, and generating sequences corresponding to the results of applying the second chemical modification combination to all positions of the nucleotide sequence of the RNA therapeutic (RNA_DRUG_SEQ), to which two or more chemical modifications included in the second chemical modification combination are applicable.

Accordingly, the number of the multiple sequences generated from the sequence modification module 300 may be much greater than w.

In addition, each of the multiple sequences may represent a molecular structure corresponding to each case in which each of the multiple chemical modification combinations is applied to the applicable positions of the nucleotide sequence of the RNA therapeutic (RNA_DRUG_SEQ).

Meanwhile, it is general that biological characteristics are better when chemical modification is applied to the positions corresponding to the bases located at one end and the bases located at the other end of the nucleotide sequence of the RNA therapeutic (RNA_DRUG_SEQ) than when chemical modification is applied to the positions corresponding to the bases in the center of the nucleotide sequence of the RNA therapeutic (RNA_DRUG_SEQ).

Therefore, in some embodiments, the sequence modification module 300 may generate the multiple sequences by applying each of the multiple chemical modification combinations to limited positions corresponding to the first a bases and the last b bases in the nucleotide sequence of the RNA therapeutic (RNA_DRUG_SEQ), wherein a and b are each an integer.

Thereafter, the sequence modification module 300 may sequentially input the multiple sequences into the optimal chemical modification prediction model, and acquire multiple output values for the multiple sequences from the optimal chemical modification prediction model.

The sequence modification module 300 may determine, as the optimal chemical modification (O_CHEM_MOD), a chemical modification combination applied to a sequence, which corresponds to the maximum value among the multiple output values, among the multiple sequences.

In addition, the sequence modification module 300 may provide the optimal chemical modification (O_CHEM_MOD) and a position in the nucleotide sequence of the RNA therapeutic (RNA_DRUG_SEQ), to which the optimal chemical modification (O_CHEM_MOD) is to be applied, based on the sequence corresponding to the maximum value among the maximum output values.

In some embodiments, the sequence modification module 300 may determine, as the optimal chemical modification (O_CHEM_MOD), all chemical modification combinations applied to sequences, which correspond to output values having a threshold value or more, among the multiple output values.

As described above with reference to FIGS. 1 to 6, the RNA therapeutic agent design system 10, 20 according to embodiments of the present invention may not only provide the nucleotide sequence of the RNA therapeutic (RNA_DRUG_SEQ), which can maximize the therapeutic effect for the specific disease by modulating the activity of target mRNA (T_MRNA) and, at the same time, effectively prevent side effects by minimizing the extent to which the RNA therapeutic modulate the activity of multiple off-target mRNAs (OFF_T_MRNA), but also provide the optimal chemical modification (O_CHEM_MOD) that may be applied to the nucleotide sequence of the RNA therapeutic (RNA_DRUG_SEQ) to further improve the therapeutic efficiency and *in vivo* stability of the RNA therapeutic.

Therefore, the RNA therapeutic agent design system 10, 20 according to embodiments of the present invention may effectively design RNA therapeutics that may have high therapeutic effects, reduced side effects, and improved *in vivo* stability.

Industrial Applicability

The present invention may be useful for designing an RNA therapeutic that may effectively modulate the activity of target mRNA while preventing the side effects of modulating the activity of other off-targeted mRNAs.

Although the present invention has been described above with reference to the preferred embodiments, those skilled in the art will understand the present invention can be variously modified and changed without departing from the spirit and scope of the present invention as set forth in the appended claims.

**Claims**

1. A method for determining an optimal chemical modification for a nucleotide sequence of an RNA therapeutic, comprising:

    a step in which a sequence modification module acquires, as learning data, data stored in a chemical modification information database that stores values representing biological characteristics which correspond to when multiple chemical modifications are applied to multiple nucleotide sequences;
    a step in which the sequence modification module creates an optimal chemical modification prediction model by repeatedly performing a process of randomly selecting, among the learning data, at least two sequences in which different chemical modifications are applied to the same nucleotide sequence, sequentially inputting the selected sequences into an artificial neural network, and training the artificial neural network to compare output values for the at least two sequences input into the artificial neural network and output a higher value as the biological characteristics for the sequence input into the artificial neural network are better;
    a step in which the sequence modification module receives a nucleotide sequence of an RNA therapeutic for modulating an activity of a target mRNA (messenger ribonucleic acid) involved in development of a specific disease; and
    a step in which the sequence modification module uses the optimal chemical modification prediction model to determine, as an optimal chemical modification, a chemical modification showing the best biological characteristics when applied to the nucleotide sequence of the RNA therapeutic, among first to $w^{th}$ chemical modifications, wherein w is an integer of 2 or more.

2. The method of claim 1, wherein the step in which the sequence modification module generates the optimal chemical modification prediction model comprises:

    a step of randomly selecting, among the learning data, a pair of a first sequence and a second sequence in which different chemical modifications are applied to the same nucleotide sequence;
    a step of sequentially inputting the first sequence and the second sequence into the artificial neural network, and acquiring a first output value for the first sequence from the artificial neural network and a second output value for the second sequence from the artificial neural network; and

a step of creating the optimal chemical modification prediction model by training the artificial neural network to increase a value obtained by subtracting the second output value from the first output value when the biological characteristics for the first sequence are better than the biological characteristics for the second sequence, and training the artificial neural network to increase a value obtained by subtracting the first output value from the second output value when the biological characteristics for the second sequence are better than the biological characteristics for the first sequence.

3. The method of claim 1, wherein the artificial neural network corresponds to a Siamese neural network.

4. The method of claim 1, wherein the values representing the biological characteristics include at least one of inhibition rate, which represents biological activity and efficacy, ED50, LD50, and IC50, which represent drug potency, ALT (alanine aminotransferase), AST (aspartate aminotransferase), bilirubin and creatinine levels, which represent drug toxicity, and elimination half-life.

5. The method of claim 1, wherein the step in which the sequence modification module uses the optimal chemical modification prediction model to determine, as the optimal chemical modification, the chemical modification showing the best biological characteristics when applied to the nucleotide sequence of the RNA therapeutic, among the first to $w^{th}$ chemical modifications comprises:

a step of generating multiple sequences by applying each of the first to $w^{th}$ chemical modifications to the nucleotide sequence of the RNA therapeutic;
a step of sequentially inputting the multiple sequences into the optimal chemical modification prediction model;
a step of acquiring multiple output values for the multiple sequences from the optimal chemical modification prediction model; and
a step of determining, as the optimal chemical modification, the chemical modification applied to a sequence, which corresponds to the maximum value among the multiple output values, among the multiple sequences.

6. The method of claim 5, wherein the step in which the sequence modification module generates the multiple sequences by applying each of the first to $w^{th}$ chemical modifications to the nucleotide sequence of the RNA therapeutic comprises a step of generating the multiple sequences by applying each of the first to $w^{th}$ chemical modifications to limited positions corresponding to the first a bases and the last b bases in the nucleotide sequence of the RNA therapeutic agent, wherein a and b are each an integer.

7. The method of claim 1, wherein the step in which the sequence modification module uses the optimal chemical modification prediction model to determine, as the optimal chemical modification, the chemical modification showing the best biological characteristics when applied to the nucleotide sequence of the RNA therapeutic, among the first to $w^{th}$ chemical modifications comprises:

a step of generating multiple chemical modification combinations by combining one or more of the first to $w^{th}$ chemical modifications;
a step of generating multiple sequences by applying each of the multiple chemical modification combinations to the nucleotide sequences of the RNA therapeutic;
a step of sequentially inputting the multiple sequences into the optimal chemical modification prediction model;
a step of acquiring multiple output values for the multiple sequences from the optimal chemical modification prediction model; and
a step of determining, as the optimal chemical modification, the chemical modification combination applied to a sequence, which corresponds to the maximum value among the multiple output values, among the multiple sequences.

8. The method of claim 7, wherein the step in which the sequence modification module generates the multiple sequences by applying each of the multiple chemical modification combinations to the nucleotide sequences of the RNA therapeutic comprises a step of generating the multiple sequences by applying each of the multiple chemical modifications to limited positions corresponding to the first a bases and the last b bases in the nucleotide sequence of the RNA therapeutic agent, wherein a and b are each an integer.

9. The method of claim 1, wherein the first to $w^{th}$ chemical modifications include at least one chemical modification that is applied to a sugar moiety in the nucleotide sequence of the RNA therapeutic, at least one chemical modification that is applied to a phosphate moiety in the nucleotide sequence of the RNA therapeutic, and at least one chemical modification that is applied to a base moiety in the nucleotide sequence of the RNA therapeutic.

10. The method of claim 1, further comprising a step in which a sequence generation module determines the nucleotide sequence of the RNA therapeutic, which modulates the activity of the target mRNA to a great extent and modulates activity of multiple off-target mRNAs other than the target mRNA to a small extent,
wherein the step in which the sequence generation module determines the nucleotide sequence of the RNA therapeutic comprises:

a step of determining a candidate nucleotide sequence;
a step of determining a reward for the candidate nucleotide sequence by increasing the reward as the extent to which the candidate nucleotide sequence modulates the activity of the target mRNA by binding to the target mRNA increases, and decreasing the reward as the extent to which the candidate nucleotide sequence modulates the activity of each of the multiple off-target mRNAs by binding to each of the multiple off-target mRNAs increases;
a step of calculating the reward while modifying the candidate nucleotide sequence in various ways, and repeatedly performing of a process of modifying the candidate nucleotide sequence in a direction that increases the reward; and
a step of determining, as the nucleotide sequence of the RNA therapeutic, the final candidate nucleotide sequence if the reward is no longer increased through the modification of the candidate nucleotide sequence.

11. The method of claim 1, further comprising:

a step in which a sequence generation module determines the nucleotide sequence of the RNA therapeutic, which modulates the activity of the target mRNA to a great extent and modulates activities of multiple off-target mRNAs other than the target mRNA to a small extent; and
a step in which a secondary structure prediction module estimates a bonding relationship between bases in the target mRNA by predicting a secondary structure in which the target mRNA is folded,
wherein the step in which the sequence generation module determines the nucleotide sequence of the RNA therapeutic comprises a step in which the sequence generation module determines, based on the bonding relationship between the bases in the target mRNA, the nucleotide sequence of the RNA therapeutic, which modulates the activity of the target mRNA to a great extent and modulates the activity of the multiple off-target mRNAs to a small extent.

12. The method of claim 11, wherein the step in which the sequence generation module determines, based on the bonding relationship between the bases in the target mRNA, the nucleotide sequence of the RNA therapeutic, comprises:

a step of determining a candidate nucleotide sequence;
a step of increasing the reward as the extent to which the candidate nucleotide sequence modulates the activity of the target mRNA by binding to the nucleotide sequence of the target mRNA increases, and decreasing the reward as the extent to which the candidate nucleotide sequence modulates the activity of each of the multiple off-target mRNAs by binding to each of the multiple off-target mRNAs increases;
a step of determining, based on the bonding relationship between the bases in the target mRNA, a secondary structure penalty proportional to a proportion of bonded bases in the target mRNA among bases of the target mRNA that binds to the candidate nucleotide sequence;
a step of determining the reward for the candidate nucleotide sequence by subtracting the secondary structure penalty from the reward;
a step of calculating the reward while modifying the candidate nucleotide sequence in various ways, and repeatedly performing a process of modifying the candidate nucleotide sequence in a direction that increases the reward; and
a step of determining, as the nucleotide sequence of the RNA therapeutic, the final candidate nucleotide sequence if the reward is no longer increased through the modification of the candidate nucleotide sequence.

13. The method of claim 1, further comprising:

a step in which a sequence generation module determines the nucleotide sequence of the RNA therapeutic, which modulates the activity of the target mRNA to a great extent and modulates activities of multiple off-target mRNAs other than the target mRNA to a small extent; and
a step in which a secondary structure prediction module predicts a secondary structure in which a corresponding mRNA is folded, for each of the target mRNA and the multiple off-target mRNAs, thereby estimating a bonding relationship between bases in the corresponding mRNA,

wherein the step in which the sequence generation module determines the nucleotide sequence of the RNA therapeutic comprises a step in which the sequence generation module determines, based on the bonding relationship between the bases in each of the target mRNA and the multiple off-target mRNAs, the nucleotide sequence of the RNA therapeutic, which modulates the activity of the target mRNA to a great extent and modulates the activity of the multiple off-target mRNAs to a small extent.

14. The method of claim 13, wherein the step in which the sequence generation module determines, based on the bonding relationship between the bases in each of the target mRNA and the multiple off-target mRNAs, the nucleotide sequence of the RNA therapeutic, comprises:

a step of determining a candidate nucleotide sequence;

a step of increasing the reward as the extent to which the candidate nucleotide sequence modulates the activity of the target mRNA by binding to the nucleotide sequence of the target mRNA increases, and decreasing the reward as the extent to which the candidate nucleotide sequence modulates the activity of each of the multiple off-target mRNAs by binding to the nucleotide sequence of each of the multiple off-target mRNAs increases;

a step of determining, based on the bonding relationship between the bases in the target mRNA, a target secondary structure penalty proportional to a proportion of bonded bases in the target mRNA among bases of the target mRNA that binds to the candidate nucleotide sequence;

a step of determining, based on the bonding relationship between the bases in the off-target mRNA for the multiple off-target mRNAs, an off-target secondary structure penalty proportional to a proportion of bonded bases in the off-target mRNA among bases of the off-target mRNA that binds to the candidate nucleotide sequence;

a step of determining the reward for the candidate nucleotide sequence by subtracting the target secondary structure penalty from the reward and adding the off-target secondary structure penalty for each of the multiple off-target mRNAs;

a step of calculating the reward while modifying the candidate nucleotide sequence in various ways, and repeatedly performing a process of modifying the candidate nucleotide sequence in a direction that increases the reward; and

a step of determining, as the nucleotide sequence of the RNA therapeutic, the final candidate nucleotide sequence if the reward is no longer increased through the modification of the candidate nucleotide sequence.

15. The method of any one of claims 10, 11 and 13, further comprising a step in which an off-target analysis module determines, as the multiple off-target mRNAs, mRNAs having gene expression patterns similar to that of the target mRNA among multiple mRNAs contained in the human body.

16. A system for determining an optimal chemical modification for a nucleotide sequence of an RNA therapeutic, comprising a sequence modification module configured to:

acquire, as learning data, data stored in a chemical modification information database that stores values representing biological characteristics which correspond to when multiple chemical modifications are applied to multiple nucleotide sequences; and

create an optimal chemical modification prediction model by repeatedly performing a process of randomly selecting, among the learning data, at least two sequences in which different chemical modifications are applied to the same nucleotide sequence, sequentially inputting the selected sequences into an artificial neural network, and training the artificial neural network to compare output values for the at least two sequences input into the artificial neural network and output a higher value as the biological characteristics for the sequence input into the artificial neural network are better,

wherein the sequence modification module, when receiving a nucleotide sequence of an RNA therapeutic for modulating an activity of a target mRNA (messenger ribonucleic acid) involved in development of a specific disease, uses the optimal chemical modification prediction model to determine, as an optimal chemical modification, a chemical modification showing the best biological characteristics when applied to the nucleotide sequence of the RNA therapeutic, among first to $w^{th}$ chemical modifications, wherein w is an integer of 2 or more.

FIG. 1

10

GE_DB —110

OFF—TARGET
ANALYSIS
MODULE

OFF_T_MRNA

~100

T_MRNA

SEQUENCE
GENERATION
MODULE

RNA_DRUG_SEQ

200

SEQUENCE
MODIFICATION
MODULE

O_CHEM_MOD

~300

CM_DB —310

FIG. 2

| Drug\\mRNA | DR1 | DR2 | ... | DRm | |
|---|---|---|---|---|---|
| MRNA1 | 1 | 0 | | −1 | V_1 |
| MRNA2 | 0 | 1 | | 0 | V_2 |
| ⋮ | | | | | |
| MRNAn | 0 | −1 | | 1 | V_n |

24

FIG. 3

C_SEQ

```
                                      G   U   U   C   G   C
                                      :   :   :   :   :   :
A   U   U   A   G   A   A   C   A   A   C   C   A   A   G   C   G
```

MRNA

→ △G

FIG. 4

20

SS_DB — 410

SECONDARY
STRUCTURE
PREDICTION
MODULE — 400

S_MAT

GE_DB — 110

OFF-TARGET
ANALYSIS
MODULE — 100

OFF_T_MRNA

T_MRNA

SEQUENCE
GENERATION
MODULE

200

RNA_DRUG_SEQ

SEQUENCE
MODIFICATION
MODULE — 300

O_CHEM_MOD

CM_DB — 310

FIG. 5

```
            G  G  G  A  A  A  C  G  U  U  C  C  G
         ┌                                          ┐
      G  │ 0  0  0  0  0  0  0  0  0  0  0  0  0 │
      G  │ 0  0  0  0  0  0  0  0  0  0  0  1  0 │
      G  │ 0  0  0  0  0  0  0  0  0  0  1  0  0 │
      A  │ 0  0  0  0  0  0  0  0  0  1  0  0  0 │
      A  │ 0  0  0  0  0  0  0  0  0  0  0  0  0 │
      A  │ 0  0  0  0  0  0  0  0  0  0  0  0  0 │
      C  │ 0  0  0  0  0  0  0  0  0  0  0  0  0 │
      G  │ 0  0  0  0  0  0  0  0  0  0  0  0  0 │
      U  │ 0  0  0  0  0  0  0  0  0  0  0  0  0 │
      U  │ 0  0  0  1  0  0  0  0  0  0  0  0  0 │
      C  │ 0  0  1  0  0  0  0  0  0  0  0  0  0 │
      C  │ 0  1  0  0  0  0  0  0  0  0  0  0  0 │
      G  │ 0  0  0  0  0  0  0  0  0  0  0  0  0 │
         └                                          ┘
```

T_MRNA                                 S_MAT

FIG. 6

A C G U G U G G C C G U U U A C G U C G C

Unmodified sequence

2-O-Methyl

A C U U G U G G C C G U U U A C G U C G C

2-O-Methoxyethyl

A C U U G U G G C C G U U U A C G U C G C

## INTERNATIONAL SEARCH REPORT

| International application No. |
|---|
| **PCT/KR2023/007219** |

**A.    CLASSIFICATION OF SUBJECT MATTER**

**G16B 40/20**(2019.01)i; **G16B 30/00**(2019.01)i; **G16B 50/00**(2019.01)i; **G16B 15/30**(2019.01)i; **G16B 30/10**(2019.01)i; **G16B 25/10**(2019.01)i; **G16B 20/00**(2019.01)i; **C12Q 1/6869**(2018.01)i; **G06N 20/00**(2019.01)i

According to International Patent Classification (IPC) or to both national classification and IPC

**B.    FIELDS SEARCHED**

Minimum documentation searched (classification system followed by classification symbols)

G16B 40/20(2019.01); G06N 20/00(2019.01); G16B 30/10(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: RNA 치료제 선택(RNA therapeutics selection), 화학적 변형(chemical modification), 인공 신경망(artificial neural network), 시퀀스 비교 반복(repeating sequence comparison), 생물학적 특성(biological feature value), 목표 mRNA(target mRNA), 비표적 mRNA(off-target mRNA)

**C.    DOCUMENTS CONSIDERED TO BE RELEVANT**

| Category* | Citation of document, with indication, where appropriate, of the relevant passages | Relevant to claim No. |
|---|---|---|
| A | DAR, Showkat Ahmad et al. SMEpred workbench: A web server for predicting efficacy of chemically modified siRNAs. RNA Biology. 2016, vol. 13, no. 11, pp. 1144-1151. See abstract; and pages 1144-1147. | 1-16 |
| A | SHAKER, Bilal et al. In silico methods and tools for drug discovery. Computers in Biology and Medicine. 2021, vol. 137, article no. 104851, pp. 1-15. See entire document. | 1-16 |
| A | CASTILLO-HAIR, Sebastian M. et al. Machine learning for designing next-generation mRNA therapeutics. Accounts of Chemical Research. 2022 (publication date: 14 December 2021), vol. 55, pp. 24-34. See entire document. | 1-16 |
| A | HUANG, Yi et al. A framework for identification of on- and off-target transcriptional responses to drug treatment. Scientific Reports. 2019, vol. 9, article no. 17603, pp. 1-9. See entire document. | 1-16 |

☑ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **24 August 2023** | **24 August 2023** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

<table>
<tr><td colspan="2" rowspan="2"><strong>INTERNATIONAL SEARCH REPORT</strong></td><td>International application No.</td></tr>
<tr><td><strong>PCT/KR2023/007219</strong></td></tr>
</table>

**C.    DOCUMENTS CONSIDERED TO BE RELEVANT**

| Category* | Citation of document, with indication, where appropriate, of the relevant passages | Relevant to claim No. |
|---|---|---|
| A | WHATLEY, Alexander et al. Improving RNA secondary structure design using deep reinforcement learning. arXiv:2111.04504v1. pp. 1-11, 05 November 2021. [Retrieved on 02 August 2023]. Retrieved from <https://arxiv.org/pdf/2111.04504.pdf>.<br>See entire document. | 1-16 |
| PX | KR 10-2499895 B1 (SPIDERCORE INC.) 16 February 2023 (2023-02-16)<br>See abstract; paragraphs [0032] and [0121]-[0137]; and claims 3-16, 18-20 and 26.<br>(* This document is the published patent of a priority application of the present international application.) | 1,4-5,9-16 |

Form PCT/ISA/210 (second sheet) (July 2022)

## INTERNATIONAL SEARCH REPORT
**Information on patent family members**

| International application No. |
|---|
| **PCT/KR2023/007219** |

| Patent document<br>cited in search report | Publication date<br>(day/month/year) | Patent family member(s) | Publication date<br>(day/month/year) |
|---|---|---|---|
| KR 10-2499895 B1 | 16 February 2023 | None | |

Form PCT/ISA/210 (patent family annex) (July 2022)